(19) Europäisches Patentamt
European Patent Office
Office européen des brevets

(11) **EP 2 072 013 A1**

(12) **EUROPEAN PATENT APPLICATION**
published in accordance with Art. 153(4) EPC

(43) Date of publication:
**24.06.2009 Bulletin 2009/26**

(21) Application number: **07807542.1**

(22) Date of filing: **19.09.2007**

(51) Int Cl.:
***A61B 8/08*** *(2006.01)*

(86) International application number:
**PCT/JP2007/068156**

(87) International publication number:
**WO 2008/044441 (17.04.2008 Gazette 2008/16)**

(84) Designated Contracting States:
**DE FR GB IT NL**
Designated Extension States:
**AL BA HR MK RS**

(30) Priority: **10.10.2006 JP 2006275956**
**23.10.2006 JP 2006287085**
**16.03.2007 JP 2007067967**

(71) Applicant: **Hitachi Medical Corporation**
**Chiyoda-ku**
**Tokyo 101-0021 (JP)**

(72) Inventor: **CHOUNO, Tomoaki**
**Tokyo 1010021 (JP)**

(74) Representative: **MERH-IP**
**Matias Erny Reichl Hoffmann**
**Paul-Heyse-Strasse 29**
**80336 München (DE)**

(54) **MEDICAL IMAGE DIAGNOSTIC APPARATUS, MEDICAL IMAGE MEASURING METHOD, AND MEDICAL IMAGE MEASURING PROGRAM**

(57)     [Objective] Providing a medical image diagnostic apparatus which can mitigate an operational burden at the time when measurement processing is performed by use of a medical image. [Means for Solution] An ultrasonic diagnostic apparatus 100 acquires input image information, which is image data of a subject, and holds it in a storage section 3. An image selection section 8 performs image recognition calculation for comparison between the input image information and past image information pieces of an image measurement database 7. When the image recognition has succeeded, a measurement-position setting section 9 refers to a record of image measurement information including a past image information piece most similar to the input image information, retrieves measurement position information of the record, and sets it. A measurement-position setting processing section 6 displays the measurement position on a display section 12 such that it is superimposed on the input image information 21. A measurement calculation section 11 performs measurement calculation for the measurement position set by the measurement-position setting section 9, and displays measurement results on the display section 12. The measurement-position setting processing section 6 updates the image measurement database 7 on the basis of the input image information, the measurement position set by the measurement-position setting section 9, etc.

FIG.1

**Description**

TECHNICAL FIELD

The present invention relates to a medical image diagnostic apparatus which measures an organ tissue by use of an image thereof.

BACKGROUND ART

In the case of a conventional medical image diagnostic apparatus, an operator designates a measurement position on an acquired image by use of an input device such as a mouse, a trackball, or the like. Subsequently, the medical image diagnostic apparatus performs calculation for measurement, and displays measurement results on a display screen.
A measurement application program is used for designation of the measurement position or point. When the operator designates a plurality of points on an image, the measurement application program measures a distance between the points, the area of a region defined by the points, or the volume of a space defined by the points. When the operator sets a predetermined region on an image, the measurement application program calculates measurement values within the region.
Main operations performed by the operator include acquiring and displaying a medical image of an object to be measured, and designating a measurement position on the medical image. These operations impose a large burden on an operator who examines a large number of medical images every day.
In order to overcome the above-described problem, there has been proposed an ultrasonic diagnostic apparatus in which setting for measurement of a fetus is automated (see Patent Document 1). This ultrasonic diagnostic apparatus holds a past measurement position of a subject patient in the form of a database. In the case of the ultrasonic diagnostic apparatus, an ID of a subject patient and an approximate measurement position in an input image are initially set. On the basis of these initially set data, the ultrasonic diagnostic apparatus determines likely measurement points from the database, and displays the measurement points on an image.
Further, there have been proposed medical image processing apparatuses which automatically set a view type of a medical image and a measurement position with reference to past medical images and measurement positions. For example, there has been proposed a medical image processing apparatus which recognizes a view type of an echocardiographic image by means of a pattern matching method (see Patent Document 2). This medical image processing apparatus holds cross-sectional images acquired in the past as dictionary images. The medical image processing apparatus performs pattern matching processing so as to determine similarity between an input image and each of the dictionary images, and uses information of the image in the database most similar to the input image.
    Patent Document 1: Japanese Patent Application Laid-Open (*kokai*) No. 2005-224465
    Patent Document 2: Japanese Patent Application Laid-Open (*kokai*) No. 2002-140689

DISCLOSURE OF THE INVENTION

PROBLEMS TO BE SOLVED BY THE INVENTION

However, designation of a measurement position requires a troublesome operation, and imposes a large burden on an operator who examines a large number of mages every day. Further, the measurement position may vary according to the personal opinions of operators, and designation of a measurement position is made difficult by poor quality of an image or little experience on the part of an operator. These problems have not yet been solved by the apparatuses described in the above-mentioned patent documents.
Further, since the conventional medical image processing apparatus premises that a medical image of a proper view type is input, if a medical image which is not a measurement subject is input, accurate measurement cannot be performed. Notably, the medical image which is not a measurement subject refers to an image which does not include an organ tissue, a medical image of an improper view type, or a medical image taken along a view deviated from a standard view.
The present invention has been accomplished in order to solve the above-described problems, and an object of the present invention is to provide a medical image diagnostic apparatus which mitigates an operational burden at the time when measurement processing is performed by use of a medical image.

MEANS FOR SOLVING THE PROBLEMS

A first invention for achieving the above-described object is a medical image diagnostic apparatus comprising image information acquiring means for acquiring image information of a subject; a display section for displaying the image information acquired by the image information acquiring means; and measurement calculation means for performing measurement calculation on the basis of the image information displayed on the display section, the apparatus being characterized by further comprising storage means for holding, in a mutually related manner, image information pieces acquired in the past and past measurement position information pieces set for the image information pieces; image selection means for recognizing and selecting a past image information piece which is most similar to the input image information; measurement-position setting means for setting a measurement position for the input image information on the basis of the past measurement position information piece corresponding to the past image information piece selected by the image selection means; and measurement position display means for displaying the measurement position set by the measurement-position setting means along with the input image information.

The medical image diagnostic apparatus of the first invention holds image information pieces acquired in the past and past measurement position information pieces set for the image information pieces in the form of a database. The medical image diagnostic apparatus performs image recognition processing in order to compare the input image information and the past image information pieces held in the database, and selects a past image information piece which is most similar to the input image information. The medical image diagnostic apparatus sets a measurement position on the basis of the past measurement position information piece corresponding to the selected past image information piece, and displays the measurement position such that the measurement position is superimposed on the input image information. The medical image diagnostic apparatus performs calculation for measurement of an organ tissue at the set measurement position, and displays the measurement results. The medical image diagnostic apparatus may be an ultrasonic diagnostic apparatus, an X-ray CT diagnostic apparatus, or an MRI diagnostic apparatus.

By virtue of the above-described configuration, the medical image diagnostic apparatus can search a past image measurement information piece on the basis of the input image information, and automatically set a measurement position, without requiring an operator to perform initial setting in advance. Thus, the operation burden of the operator can be mitigated.

The image recognition calculation may be performed for comparison between the input image information and all the past image information pieces held in the storage means.

By virtue of this configuration, an optimal measurement position can be set on the basis of all the past image information pieces and the measurement position information pieces.

The medical image diagnostic apparatus may be configured as follows. The past image information pieces are classified into a plurality of categories, and representative image information which represents image information pieces belonging to each category is held in the database. The image recognition calculation is first performed so as to compare the input image information and the representative image information to thereby determine the category of the input image information. Subsequently, the image recognition calculation is performed so as to compare the input image information and past image information pieces belonging to the determined category.

By virtue of this configuration, the medical image diagnostic apparatus first performs rough classification and then fine classification for the input image information. Therefore, the recognition ratio and the processing efficiency are improved.

Further, since the medical image diagnostic apparatus can recognize the category of the input image information, classification and measurement of the input image information can be performed simultaneously, and the measurement results can be automatically classified for arranging them in order.

The medical image diagnostic apparatus may be configured as follows. Past measurement condition information pieces are held in the database while being related to the past image information pieces. Measurement condition recognition calculation is performed so as to compare input measurement condition information and the past measurement condition information pieces, and a past measurement condition information piece most similar to the input measurement condition information is recognized and selected. The measurement position is set on the basis of a past measurement position information piece corresponding to the selected past measurement condition information piece.

The measurement condition is various conditions associated with measurement. Examples of the measurement condition include not only initially set conditions and measurement items, but also measurement timing, time-series frame numbers, the age and sex of a subject, diagnostic information regarding the subject, and the category of the to-be-measured object or the subject.

By virtue of this configuration, the medical image diagnostic apparatus can search a past image measurement information piece on the basis of not only the input image information but also the input measurement condition, and automatically set a measurement position. The medical image diagnostic apparatus can set an accurate measurement position in accordance with not only the input image information but also the input measurement condition. For example, the medical image diagnostic apparatus can automatically change the determined measurement position to a measurement position corresponding to a measurement item selected by the operator.

Preferably, the medical image diagnostic apparatus is configured to determine whether or not the set measurement position is proper on the basis of an image feature quantity of the input image information, and to correct the set measurement position on the basis of the image feature quantity of the input image information when the set measurement position is not proper.

Further, the medical image diagnostic apparatus may be configured to calculate an edge intensity in the vicinity of the measurement position set for the input image information, and to correct the set measurement position to a position where the calculated edge intensity becomes the maximum.

Further, the medical image diagnostic apparatus may be configured to correct the set measurement position on the basis of a discrepancy between image information in the vicinity of the measurement position set for the input image information and image information in the vicinity of the measurement position indicated by the past measurement position information.

By virtue of these configurations, even in the case where a past image information piece similar to the input image information is not present in the database and the set measurement position is not proper, the measurement position can be corrected on the basis of the input image information. Further, the medical image diagnostic apparatus may be configured such that when the corrected measurement position is not proper, the operator can manually correct the measurement position.

The medical image diagnostic apparatus may be configured to set a region of interest on the basis of the set measurement position and display it along with the input image information.

By virtue of this configuration, when the input image information is displayed, a region of interest is automatically set, and measurement results regarding the region of interest are displayed. Since not only the measurement point but also the region of interest is automatically set, the operation burden can be mitigated, and the time required for diagnosis can be shortened.

The medical image diagnostic apparatus may be configured to select an image portion of a predetermined range from the input image information, the range including the set measurement position, and track a movement of the image portion to thereby move the set measurement position.

By virtue of this configuration, the medical image diagnostic apparatus sets a measurement point(s) for a frame image captured at a predetermined point in time on the basis of the past image information and measurement position information, and tracks the measurement point(s) for the next and subsequent frame images on the basis of the degree of coincidence between the images. Accordingly, the medical image diagnostic apparatus can automatically set the measurement position, and move the measurement point(s) in accordance with movement of a relevant tissue, to thereby accurately measure the tissue dynamics (movement of a relevant tissue such as the cardiac muscle).

The medical image diagnostic apparatus may be configured to display a result of the image recognition associated with the input image information when the image recognition has succeeded.

By virtue of this configuration, the operator can confirm the view type or an organ tissue portion currently displayed on the screen.

The medical image diagnostic apparatus may be configured to perform warning processing or processing for inputting the input image information again when the image recognition has failed.

By virtue of this configuration, when the image recognition is impossible, the medical image diagnostic apparatus can prompt the operator to acquire the input image information again or automatically acquire the input image information again.

Preferably, the medical image diagnostic apparatus is configured to register the newly input image information and a measurement position set for the input image information in the database for update.

By virtue of this configuration, the amount of information of the database increases, and a learning effect can be attained; i.e., the image recognition ratio can be improved when image information is newly input at the next time.

The medical image diagnostic apparatus may be configured as follows. Brightness information pieces of a plurality of cross-sectional images acquired in the past and their view types are held in the storage means in a mutually related manner. The medical image diagnostic apparatus calculates a similarity between the input image and the plurality of cross-sectional images held in the storage means, and calculates a similarity difference which is a difference between the similarity between the input image and a cross-sectional image which is most similar to the input image and the similarity between the input image and anther cross-sectional image. The medical image diagnostic apparatus then performs threshold processing for the similarity and the similarity difference so as to determine whether or not the input image is to be rejected.

By virtue of this configuration, when the input image resembles two standard cross-sectional images and is vague, the medical image diagnostic apparatus can reject the input image and prompt the operator to again acquire an input image of a view type necessary for measurement. Accordingly, the processing can be performed quickly and efficiently.

The medical image diagnostic apparatus may be configured to calculate a brightness statistic of the input image and then perform the threshold processing so as to determine whether or not the input image includes a statistical characteristic peculiar to an organ tissue image of a measurement subject, to thereby determine whether or not the input

4

image is to be rejected.

Since the statistical characteristic of the input image is used as a reject criterion, an input image which does not include an organ tissue of a measurement subject can be rejected.

The medical image diagnostic apparatus may be configured such that a reject ratio or a false recognition ratio is set in place of the threshold value, and the threshold processing is performed by use of a threshold value corresponding to the reject ratio or the false recognition ratio so as to determine whether or not the input image is to be rejected.

By virtue of use of the reject ratio or the false recognition ratio in place of the threshold value, it becomes possible to adapt the operation feeling of the operator.

The medical image diagnostic apparatus may be configured to determine a range or the number of view types used as classification categories on the basis of a set measurement item.

By virtue of selecting view types used as classification categories in accordance with the measurement item, the recognition ratio can be increased, and the operability in the measurement operation can be improved.

A second invention is a medical image measurement method for acquiring image information of a subject, displaying the acquired image information, and performing measurement calculation on the basis of the displayed image information, the method characterized by comprising a step of holding, in a storage apparatus, image information pieces acquired in the past and past measurement position information pieces set for the image information pieces such that the image information pieces and the measurement position information pieces are related to each other; an image selection step of recognizing and selecting a past image information piece which is most similar to the input image information; a measurement-position setting step of setting a measurement position for the input image information on the basis of the past measurement position information piece corresponding to the past image information piece selected by the image selection step; and a measurement position display step of displaying the measurement position set by the measurement-position setting step along with the input image information.

The second invention relates to a medical image measurement method for acquiring image information of a subject, displaying the acquired image information, and performing measurement calculation on the basis of the displayed image information.

EFFECTS of THE INVENTION

According to the present invention, it is possible to provide a medical image diagnostic apparatus which mitigates an operational burden at the time when measurement processing is performed by use of a medical image.

BRIEF DESCRIPTION OF THE DRAWINGS

FIG. 1 is a diagram showing the configuration of an ultrasonic diagnostic apparatus 100.
FIG. 2 is a diagram showing an example of an image measurement database 7.
FIG. 3 is a flowchart showing operation of a measurement-processing setting processing section 6.
FIG. 4 is a diagram showing an example of a display screen 31 of a display section 12.
FIG. 5 is a pair of diagrams showing examples of a display screen 34 of the display section 12.
FIG. 6 is a diagram showing an example of the image measurement database 7.
FIG. 7 is a flowchart showing operation of the measurement-processing setting processing section 6.
FIG. 8 is a diagram showing an example of the image measurement database 7.
FIG. 9 is a flowchart showing operation of the measurement-processing setting processing section 6.
FIG. 10 is a flowchart showing operation of a measurement-position correction section 10.
FIG. 11 is a pair of diagrams showing examples of a display screen 51 in measurement-position correction processing.
FIG. 12 is a diagram showing an example of the display screen 51 in measurement-position correction processing.
FIG. 13 is a diagram showing setting of a region of interest 79 in an ultrasonic image 61.
FIG. 14 is a set of diagrams showing tracking of measurement points in an ultrasonic image.
FIG. 15 is a diagram showing the configuration of an ultrasonic diagnostic apparatus 150.
FIG. 16 is a diagram showing an example of a record held in an image brightness database 171.
FIG. 17 is a flowchart showing reject processing based on an image-brightness statistic of an input image.
FIG. 18 is a diagram showing a screen 228 and an ultrasonic image 229 displayed on the display section 12.
FIG. 19 is a flowchart showing reject processing based on similarity.
FIG. 20 is a diagram showing the similarity between an input image 220 and a standard cross-sectional image for each view type.
FIG. 21 is a flowchart showing reject processing based on similarity difference.
FIG. 22 is a diagram showing the similarity between an input image 220 and a standard cross-sectional image for each view type.

FIG. 23 is a diagram showing an example of a screen 250 displayed by the display section 12.

FIG. 24 is a pair of diagrams showing a screen 260 and a screen 264 displayed on the display section 12.

FIG. 25 is a flowchart showing reject processing based on similarity.

FIG. 26 is a diagram showing view types of the heart.

FIG. 27 is a diagram showing measurement items and classification categories.

DESCRIPTION OF REFERENCE NUMERALS

100: ultrasonic diagnostic apparatus

1: ultrasonic probe

2: image generation section

3: storage section

4: operation section

5: measurement-item setting section

6: measurement-position setting processing section

7: image measurement database

8: image selection section

9: measurement-position setting section

10: measurement-position correction section

11: measurement calculation section

12: display section

13: measurement condition selection section

14: tracking section

21: input image information

22: image measurement information

23: measurement position information

26, 28: image information around measurement position

24: image information

25: measurement condition information

31, 34, 51: display screen

32, 35, 52: image

33, 37: measurement point

36: measurement item

38: measurement region

41: classified image measurement database

42: representative image information

43: input measurement condition

53, 83, 88: endocardial contour

54, 55, 57: measurement point before correction

56, 58: measurement point after correction

61, 81, 86: ultrasonic image

62: carotid artery

63, 64: wall surface

71 - 78: measurement point

79: region of interest

82, 87: cardiac muscle

84: measurement point before being moved

85: cutout image

89: local image

91: measurement point after being moved

150: ultrasonic diagnostic apparatus

160: image-brightness extraction section

170: view recognition processing section

171: image brightness database

172: similarity calculation section

173: view-type determination section

180: reject processing section

181: image-brightness-statistic calculation section

182: similarity-difference calculation section

183: threshold setting section

184: reject determination section

220: input image

221: view type

222: standard cross-sectional image information

223: measurement item

224: measurement position

228, 250, 260, 264: screen

229, 251, 262, 266: ultrasonic image

252: display of view type

253: display of similarity

254: display of graph

261, 265, 281, 283: measurement item

263: measurement point

267: measurement region

270: heart

271 - 276: standard cross-sectional image

BEST MODE FOR CARRYING OUT THE INVENTION

A preferred embodiment of a medical image diagnostic apparatus according to the present invention will be described in detail with reference to the attached drawings. Notably, in the following description and the attached drawings, components having generally the same functions are denoted by like reference numerals, and their descriptions will not be repeated.

The medical image diagnostic apparatus according to the present invention can be applied to an ultrasonic diagnostic apparatus, an X-ray CT diagnostic apparatus, an MRI diagnostic apparatus, or a system in which these apparatuses are combined. In the following description, there will be described measurement based on a cross-sectional image of the heart will be described, which measurement is performed by an ultrasonic diagnostic apparatus, which is an example medical image diagnostic apparatus. Notably, the present invention can be applied to organ tissues other than the heart. Further, the present invention can be applied to images other than cross-sectional images.

(1. Configuration of an ultrasonic diagnostic apparatus)

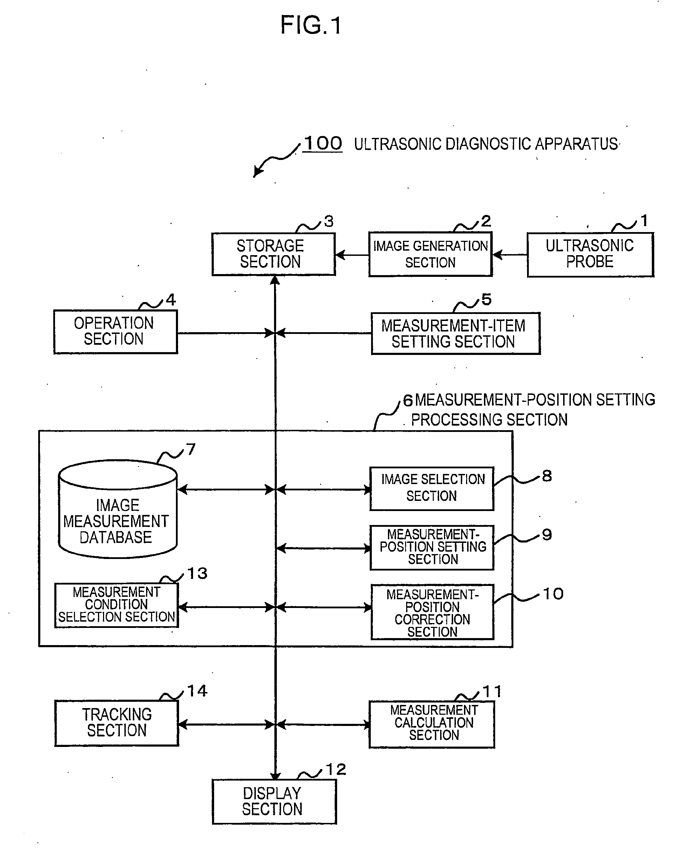

First, the configuration of an ultrasonic diagnostic apparatus 100 will be described with reference to FIG. 1. FIG. 1 is a diagram showing the configuration of the ultrasonic diagnostic apparatus 100.

The ultrasonic diagnostic apparatus 100 comprises an ultrasonic probe 1 for transmitting and receiving ultrasonic waves; an image generation section 2 for generating an image from an ultrasonic signal; a storage section 3 which serves as a storage area for storing the image; an operation section 4 for enabling an operator to operate the apparatus by use of input devices; a measurement-item setting section 5 for setting measurement items; a measurement-position setting processing section 6 for automatically setting a measurement position; a measurement calculation section 11 for performing measurement calculation while using the measurement position; a display section 12 for displaying the measurement position and measurement results; and a tracking section 14.

The measurement-position setting processing section 6 comprises an image measurement database 7 for storing at least image information, a measurement position, and image information of a region in the vicinity of the measurement position, which were used in the past; an image selection section 8 for selecting an image by performing image recognition processing while using input image information and the image measurement database 7; a measurement-position setting section 9 for setting a measurement position corresponding to the recognized image; a measurement-position correction section 10 for evaluating and correcting the position designated by the measurement-position setting section 9; and a measurement condition selection section 13 for selecting a measurement condition by performing measurement condition recognition processing while using input measurement conditions and the image measurement database 7.

The ultrasonic probe 1 is a device for transmitting and receiving ultrasonic waves to and from a subject. The ultrasonic probe 1 may assume a sector shape, a linear shape, a convex shape, or a like shape. The ultrasonic probe 1 receives an ultrasonic wave reflected from the subject, converts it to an electric signal, and inputs the electric signal to the image generation section 2.

The image generation section 2 generates a B-mode image on the basis of the input signal from the ultrasonic probe 1. The input signal is converted to a B-mode image though a phasing adder, a logarithmic amplifier, an envelop detector, an A/D converter, and a scan converter.

The storage section 3 stores acquired images. For example, the storage section 3 may be a hard disk, a general purpose memory, a frame memory, or a cine memory.

The operation section 4 includes input devices such as a keyboard, a mouse, a trackball, etc. The operator adjusts image quality, instructs measurement, and inputs information through the operation section 4.

The measurement-item setting section 5 selects a measurement item demanded by the operator. Examples of the measurement item include a distance, volume, myocardial wall thickness, and Doppler of a certain portion.

The measurement-position setting processing section 6 sets a measurement position by performing image recognition processing while using the input image information and the database.

The image measurement database 7 stores an aggregate of records each including measurement position information, image information, and image information of a region in the vicinity of the measurement position, which were used in the past. Notably, in order to reduce the data volume, these pieces of information may be held after compression coding. Further, the image measurement database 7 holds measurement condition information while relating it to the image information. The measurement condition information will be described later.

The image selection section 8 performs image recognition calculation for the input image information by use of the image measurement database 7. The image selection section 8 selects, from the image measurement database 7, an image information piece which is most similar to the input image information by performing the image recognition calculation. Notably, pattern matching calculation such as correlation calculation can be used as the image recognition calculation. The measurement condition selection section 13 performs measurement-condition recognition calculation for the input measurement condition on the basis of the image measurement database 7. The measurement condition selection section 13 selects, from the image measurement database 7, a measurement condition information piece which is most similar to the input measurement condition by performing the measurement-condition recognition calculation.

The measurement-position setting section 9 sets a measurement position on the basis of measurement position information contained in a record of image information selected from the image measurement database 7. Notably, desirably, the measurement-position setting section 9 sets the measurement position while selecting information pieces needed for the measurement item designated by the measurement-item setting section 5.

The measurement-position correction section 10 corrects the measurement position set by the measurement-position setting section 9. The measurement-position correction section 10 evaluates the measurement position and determines whether or not the set measurement position is proper. When the set measurement position is not proper, the measurement-position correction section 10 corrects the measurement position.

For evaluation of the measurement position, for example, an edge intensity may be used as an evaluation value. For correction of positional shifts, for example, an edge detection method may be used. Further, the correction may be performed by use of a discrepancy between the image information of a region in the vicinity of the measurement position indicated by the measurement position information of the image measurement database 7 and the image information of a region in the vicinity of the set measurement position. Moreover, the measurement position may be manually corrected via the input devices.

The measurement calculation section 11 performs various measurements by use of the set measurement position. Examples of the measurements performed by the measurement calculation section 11 include calculation of a distance and a volume, measurement based on a Doppler method, stress echo, formation of a time-intensity curve in a contrast media mode, and strain measurement.

The display section 12 is composed of a display apparatus such as a CRT display or a liquid-crystal display. The display section 12 displays the set measurement position on the input image in a superimposed manner, displays the recognition results, or displays the results of the measurement calculation.

The tracking section 14 follows a motion of an organ tissue in a moving image displayed on the display section 12.

(2. First embodiment)

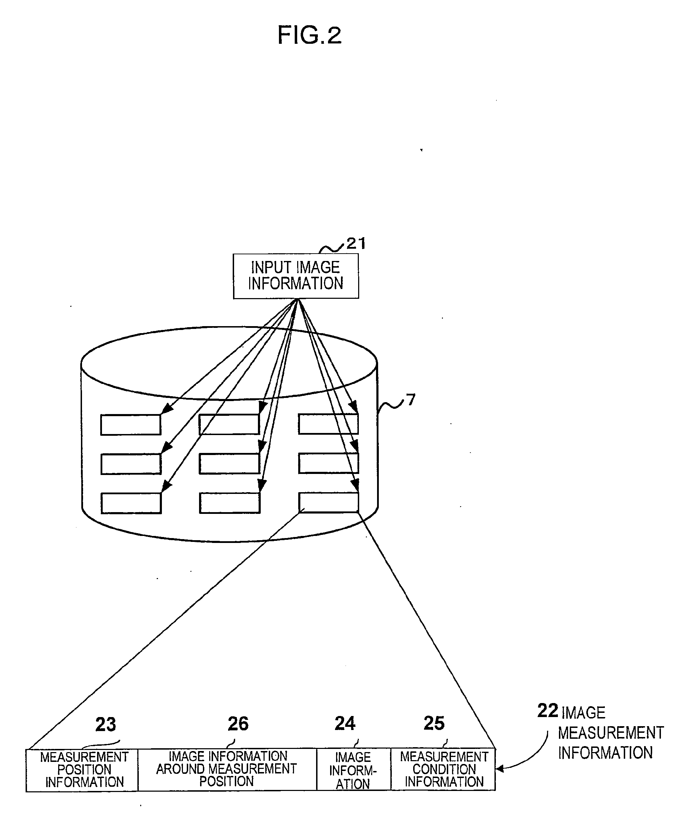

Next, an operation of the measurement-position setting processing section 6 according to a first embodiment will be described with reference to FIGS. 2 to 5.

(2-1. Image measurement database 7)

FIG. 2 is a diagram showing an example of the image measurement database 7.

The image measurement database 7 holds a plurality of records of image measurement information 22. The image measurement information 22 is information regarding an image and a measurement position which were used for measurement in the past. The image measurement information 22 includes measurement position information 23, image information 24, and measurement condition information 25, which are related to a certain single measurement and are held as a single record. Further, the image measurement information 22 may include image information 26 of a region around the measurement position indicated by the measurement position information 23.

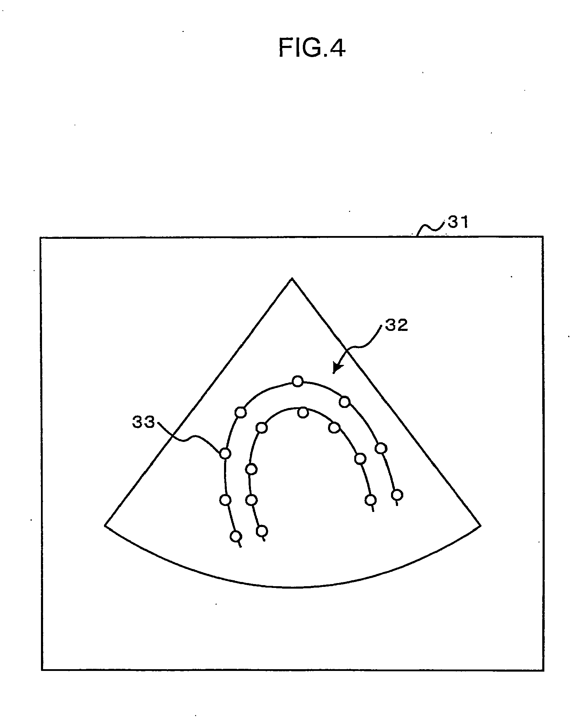

The measurement position information 23 is information regarding a measurement position; for example, coordinate values representing measurement points or grouped contour points which define a measurement region. The measurement position information 23 will be described with reference to FIG. 4, in which an image 32 and measurement points 33 are displayed in a superimposed manner on a display screen 31 of the display section 12. On the image 32 used for a past measurement, a plurality of measurement points 33 are set, for example, along the wall of the heart. The plurality of measurement pints 33 are the above-described grouped contour points which defines a measurement region. The coordinate values of these measurement points 33 represented by X-Y coordinates; i.e., (X1, Y1), (X2, Y2), etc., are held in the image measurement database 7 as the measurement position information 23. Notably, in the present embodiment, the coordinate values in the X-Y coordinate system are held in the image measurement database 7. However, the measurement position information 23 of the plurality of measurement points 33 may be held in the image measurement database 7 by use of their coordinate values in a r-θ coordinate system.

The image information around measurement position 26 is image information of a region around the measurement position indicated by the measurement position information 23. The image information around measurement position 26 includes, for example, brightness values of a region around each measurement point or brightness values of a region around each of the grouped contour points which define a measurement region. The brightness values of a region around each measurement point refers to brightness values of a square region which extends around the measurement point and whose sides have a length of 1 cm to 2 cm. Notably, this region may be an oval region or a circular region. The image information 24 is information regarding the image 32 of FIG. 4, for example, brightness values of the image 32 itself, or compression-coded brightness values. The image 32 used in the past measurement has different brightness values at different (X, Y) coordinates. Therefore, the brightness values of the entire image 32 are held in the image measurement database 7 as the image information 24, with the coordinate values being related to the brightness values. The compression-coded brightness value refers to, for example, a compressed brightness value of each scan line or a compressed brightness value of an arbitrarily selected one of two pixels. Since the coordinate values are stored while being related to the compressed brightness values, the data volume of the records held in the image measurement database 7 can be reduced. Further, the data volume can be reduced by performing principal component analysis on the entire image 32 or the above-described compressed brightness values and storing only the principal component which characterizes the nature of the brightness values.

Notably, in the present embodiment, coordinate values in the X-Y coordinate system and corresponding brightness values are held in the image measurement database 7. However, coordinate values in the r-θ coordinate system and corresponding brightness values may be held in the image measurement database 7.

The measurement condition information 25 is information regarding a measurement environment and an object to be measured. For example, the measurement condition information 25 includes initially set conditions, measurement items, measurement timing, time-series frame numbers, the age and sex of a subject, diagnostic information regarding the subject, and the category of the to-be-measured object or the subject.

(2-2. Operation of the measurement-position setting processing section 6)

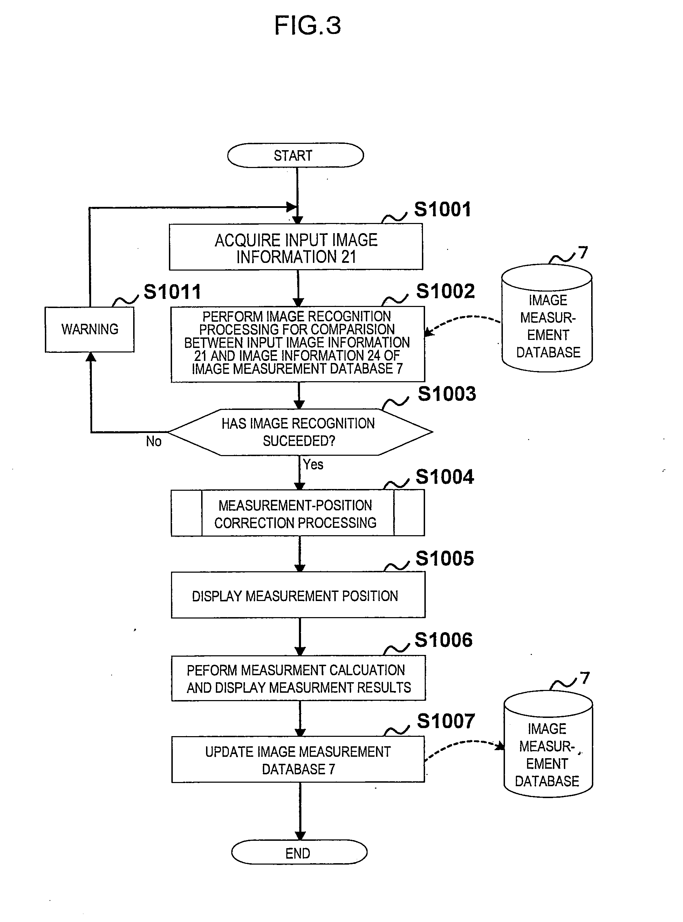

FIG. 3 is a flowchart showing operation of the measurement-processing setting processing section 6.

The ultrasonic diagnostic apparatus 100 acquires input image information 21, which is a set of image data of a subject, and holds it in the storage section 3. Image data generated by the image generation section 2 while using the ultrasonic probe 1 may be input as the input image information 21. Alternatively, image data generated by a different medical image diagnostic apparatus may be input as the input image information 21 (S1001).

The image selection section 8 performs image recognition calculation for comparison between the input image information 21 and the image information 24 of the image measurement database 7 (S1002). Pattern matching calculation such as correlation calculation can be used as the image recognition calculation. For example, the image selection section 8 obtains a correlation value by comparing (performing correlation calculation or similarity calculation for) the input image information 21 and the plurality of pieces of image information 24 whose brightness values are stored while being related to the coordinate values. Subsequently, the image selection section 8 determines whether or not the correlation value is greater than a predetermined threshold value.

The image selection section 8 then determines whether or not the image recognition calculation has been successfully completed (S1003). For example, the image selection section 8 determines that the image recognition has succeeded when the value (correlation coefficient or similarity) obtained through the recognition calculation exceeds a predetermined threshold value, and determines that the image recognition has failed when the value obtained through the recognition calculation does not exceed the predetermined threshold value. The image selection section 8 then selects a piece of the past image information 24, which is most similar to the input image information 21.

When the image recognition has succeeded (Yes in S1003), the measurement-position setting section 9 retrieves one record of the image measurement information 22 having the image information 24 which is most similar to the input image information 21. In the record, the measurement position information 23, the image information 24, and the measurement condition information 25, and the image information 26 around the measurement position are related to one another. The measurement-position setting section 9 extracts and sets the measurement position information 23 contained in the record of the image measurement information 22.

Further, the measurement-position setting section 9 extracts information corresponding to a predetermined measurement item from the measurement position information 23. Specifically, examples of the measurement items include four-chamber, two-chamber, long-axis, short-axis (base level), short-axis (mid level), and short-axis (apex level). The measurement position information 23 (coordinate values representing measurement points and grouped contour points which define a measurement region) is previously determined for each measurement item. For example, for the two-chamber, the measurement position information 23 is determined such that the measurement points are arranged to form a U-shaped curve which convexly curves upward in the vicinity of the center of the image 32. Further, for the short-axis (apex level) image, the measurement position information 23 is determined such that the measurement points are arranged to form a circle in the vicinity of the center of the image 32. The operator can select a measurement item through operation of the measurement-item setting section 5, and the measurement-position setting section 9 reads the measurement position information 23 stored while being related to the measurement item, to thereby extract the measurement position information 23 corresponding to the measurement item.

The measurement-position correction section 10 corrects the measurement position information 23 retrieved by the measurement-position setting section 9 on the basis of the input image 21 (S1004). Notably, the details of the measurement-position correction processing will be described later.

The measurement-position setting processing section 6 sets a measurement position on the basis of the measurement position information 23 having been corrected. The measurement-position setting processing section 6 displays the measurement position on the display section 12 such that the measurement position is superimposed on the input image information 21 (S1005). Notably, the measurement-position setting processing section 6 desirably performs initial setting on the basis of the measurement condition information 25 of the image measurement information 22.

The measurement calculation section 11 performs measurement calculation for the measurement position set by the measurement-position setting section 9, and displays the measurement results on the display section 12 (S1006). The measurement-position setting processing section 6 updates the image measurement database 7 on the basis of the input image information 21, the measurement position set by the measurement-position setting section 9, and the like (S1007).

Notably, when the image recognition has succeeded (Yes in S1003), the measurement-position setting processing section 6 displays an image recognition result on the display section 12. For example, the view type or an organ tissue portion currently displayed on the screen is displayed as the image recognition result.

Meanwhile, when the image recognition has failed (No in S1003), the measurement-position setting processing section 6 warns the operator to acquire the input image information 21 again (S1011). The warning can be made through display of a warning on the display section 12 or generation of a sound. Further, the measurement-position setting processing section 6 may be configured so as to automatically repeat the processing which starts from S1001.

FIG. 4 is a diagram showing an example of a display screen 31 of the display section 12.

On the display screen 31, the image 32 and the measurement points 33 are displayed in a superimposed manner. The image 32 is produced from the input image information 21. The image 32 is an echocardiographic image. The measurement points 33 show measurement positions. The positions of the measurement points 33 are set on the basis of the corrected measurement position information 23.

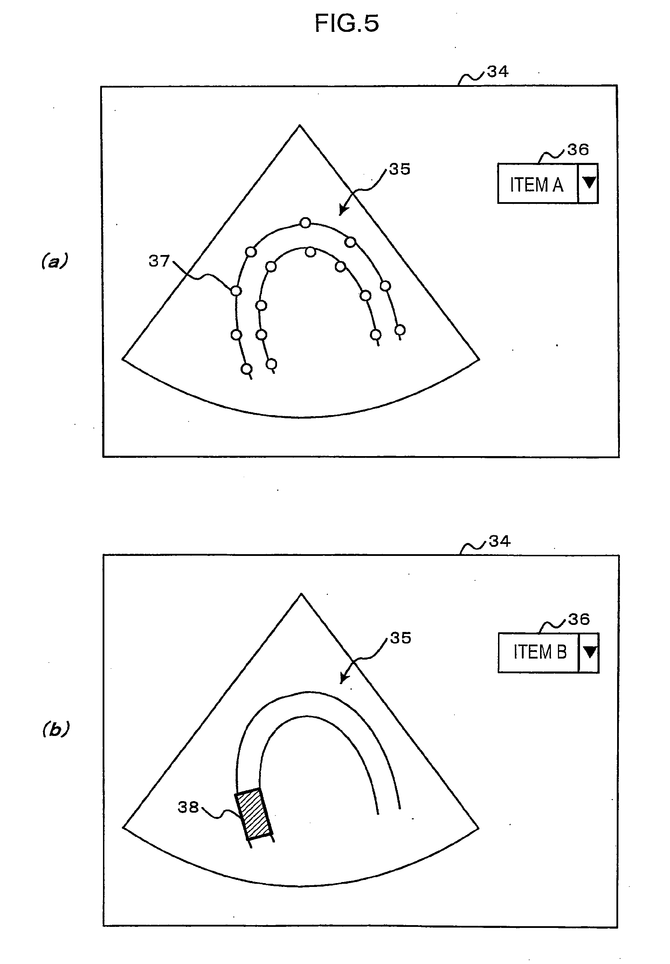

FIG. 5 is a pair of diagrams showing examples of a display screen 34 of the display section 12.

When the operator selects an "item A" on the display screen 34 as an measurement item 36 as shown in FIG. 5(a), measurement points 37 corresponding to the measurement item are displayed such that the measurement points 37 are superimposed on the image 35. When the operator selects an "item B" on the display screen 34 as the measurement item 36 as shown in FIG. 5(b), a measurement region 38 corresponding to the measurement item is displayed such that the measurement region 38 is superimposed on the image 35.

Through the above-described process, the measurement-position setting processing section 6 performs one-to-one image recognition calculation on the basis of the image measurement information 22 of the database 7 while using the input image information 21 as a key. The measurement-position setting processing section 6 searches from the image measurement database 7 the image measurement information 22 whose image information 24 is most similar to the input image information 21. The measurement-position setting processing section 6 sets a measurement position on the input image information 21 on the basis of the past measurement-position information 23 contained in the searched record of the image measurement information 22.

As described above, the ultrasonic diagnostic apparatus of the first embodiment can search past image measurement information on the basis of the input image information and automatically set a measurement position, without requiring the operator to perform initial setting in advance. Further, the ultrasonic diagnostic apparatus can automatically switch the set measurement position to a measurement position corresponding to a measurement item selected by the operator. Moreover, through speeding up of the image recognition calculation, the ultrasonic diagnostic apparatus can update the measurement position real-time in response to an operation of the ultrasonic probe 1 by the operator.

The ultrasonic diagnostic apparatus holds, in a storage apparatus, past measurement position information and image information (e.g., brightness information) without relating them to subject patients. The ultrasonic diagnostic apparatus performs image recognition processing for comparison with the image information within the database while using the input image information as a key, without using a patient ID or a rough measurement position as a key. Thus, the ultrasonic diagnostic apparatus can set the measurement position without requiring the operator to perform initial setting.

(3. Second embodiment)

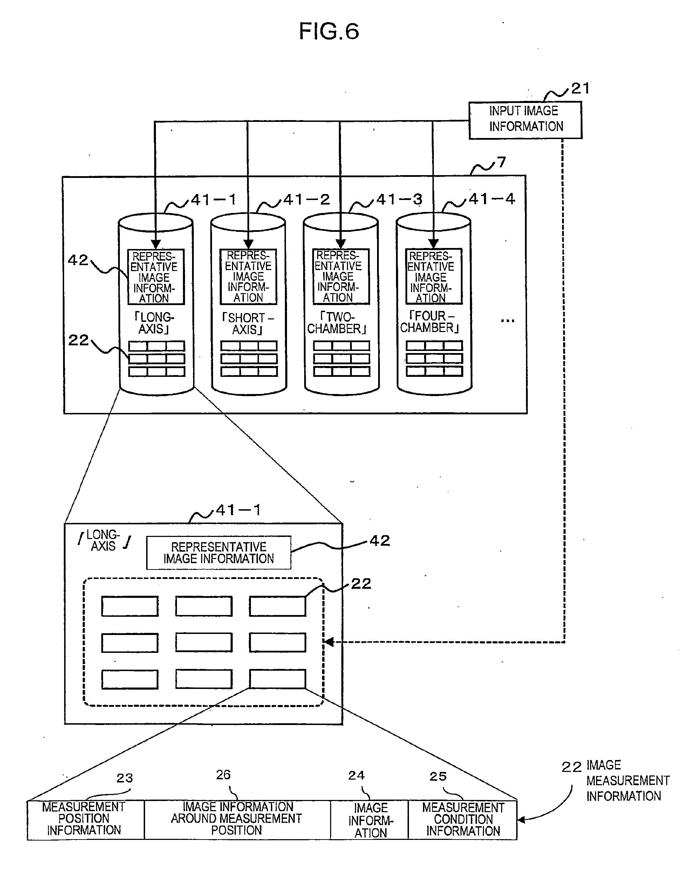

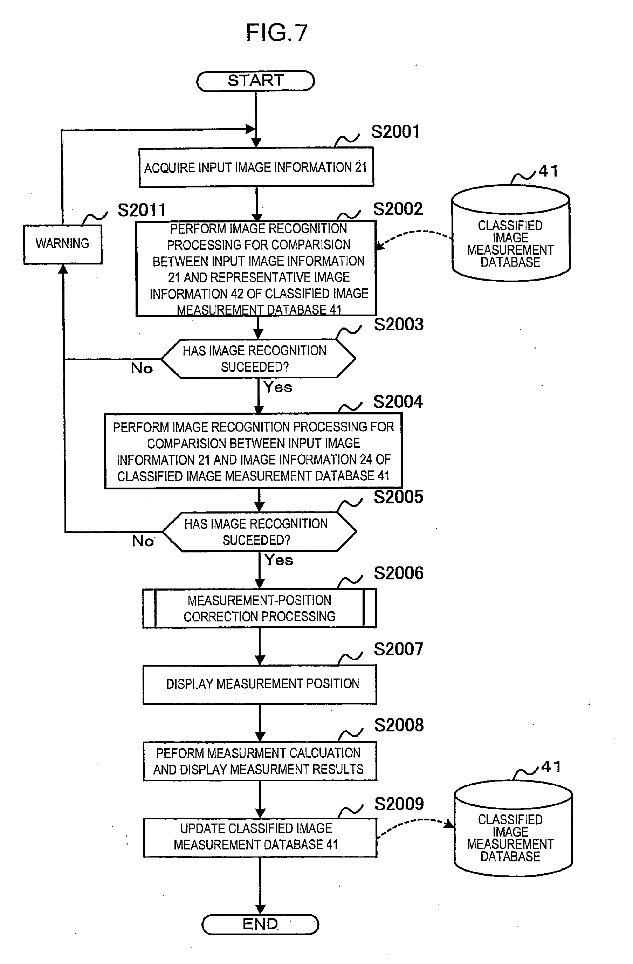

Next, a second embodiment will be described with reference to FIGS. 6 and 7.
In the first embodiment, the one-to-one image recognition calculation is performed for comparison between the input image information 21 and the image information 24 of the image measurement database 7. In the second embodiment, image recognition calculation is performed so as to compare the input image information 21 with representative image information 42, which represents images of each category, before performance of the one-to-one image recognition calculation for comparison between the input image information 21 and the image information 24.

(3-1. Image measurement database 7)

FIG. 6 is a diagram showing an example of the image measurement database 7.
The image measurement database 7 holds a plurality of categorized image measurement databases 41-1, 41-2, etc. The categorized image measurement databases 41-1, 41-2, etc. each hold the representative image information 42 and a plurality of records of the image measurement information 22.

Each of the categorized image measurement databases 41-1, 41-2, etc. is a database which holds the image measurement information 22 classified to a predetermined category. The predetermined category may be a category regarding the subject or the object to be measured. Examples of the categories include the measurement items ("long-axis," "short-axis," "two-chamber," "four-chamber," etc.), the age and sex of the subject, and the type of a disease. The predetermined category is held in the field of the measurement condition information 25 of the image measurement information 22. For example, the categorized image measurement database 41-1 holds the image measurement information 22 classified to the "long-axis" image.

The representative image information 42 is image information which represents image information pieces of the corresponding category. Each of the categorized image measurement databases 41-1, 41-2, etc. holds at least one piece of the representative image information 42. The representative image information 42 is created on the basis of the image measurement information 22 held in the corresponding categorized image measurement database 41-1, 41-2, etc.

(3-2. Operation of the measurement-position setting processing section 6)

FIG. 7 is a flowchart showing operation of the measurement-processing setting processing section 6.
The ultrasonic diagnostic apparatus 100 acquires input image information 21, which is a set of image data of a subject, and holds it in the storage section 3 (S2001).

The image selection section 8 performs image recognition calculation for comparison between the input image information 21 and the representative image information pieces 42-1, 42-2, etc. of the categorized image measurement databases 41-1, 41-2, etc. (S2002). Pattern matching calculation such as correlation calculation can be used as the image recognition calculation. The image selection section 8 then determines whether or not the image recognition calculation has been successfully completed (S2003).
When the image recognition has succeeded (Yes in S2003), the image selection section 8 classifies the input image information 21 to a category to which the representative image information 42 most similar to the input image information 21 belongs.

The processing in S2004 to S2009 and S2011 is identical with the processing in S1002 to S1007 and S1011 of FIG. 3, except that the categorized image measurement database 41 is used rather than the image measurement database 7.
Notably, the input image information 21 is held in the categorized image measurement database 41 determined through the processing in S2002 to S2003. Further, the measurement-position setting processing section 6 may be configured to create a new piece of the representative image information 42 on the basis of the input image information 21 and the existing image information 24, and store it in the categorized image measurement database 41.

Through the above-described process, the measurement-position setting processing section 6 first performs image recognition calculation for comparison between the input image information 21 and the representative image information 42 which represents image information pieces of each category, to thereby determine the category of the input image information 21. Subsequently, the measurement-position setting processing section 6 performs one-to-one image recognition calculation on the basis of the image measurement information 22 of the categorized image measurement database 41 of the determined category while using the input image information 21 as a key. Notably, each category may be divided into a plurality of sub-categories. In this case, processing of three or more stages is performed.

As described above, the ultrasonic diagnostic apparatus of the second embodiment performs rough classification and then fine classification for the input image information. Therefore, the recognition ratio and the processing efficiency are improved. Further, since the ultrasonic diagnostic apparatus can recognize the category of the input image information, the classification and measurement of the input image information can be performed simultaneously, and measurement results can be automatically classified for arranging them in order.

(4. Third embodiment)

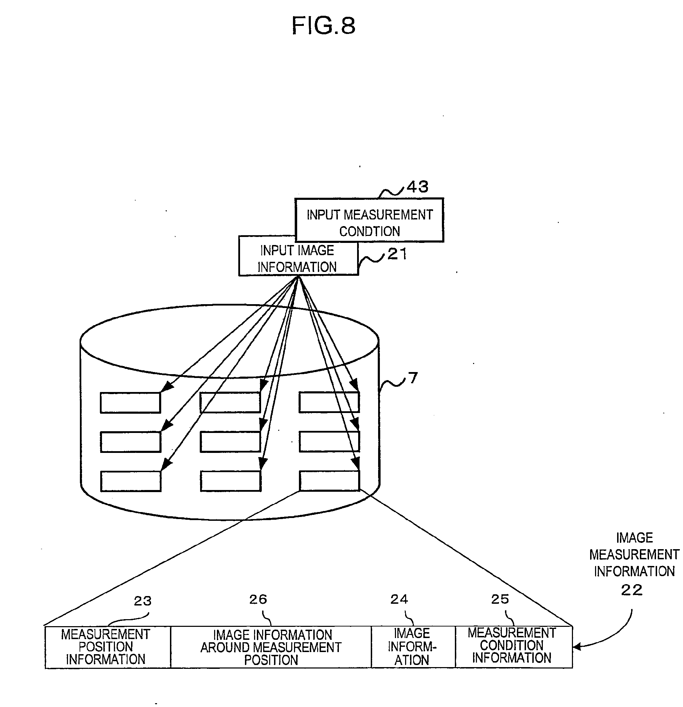

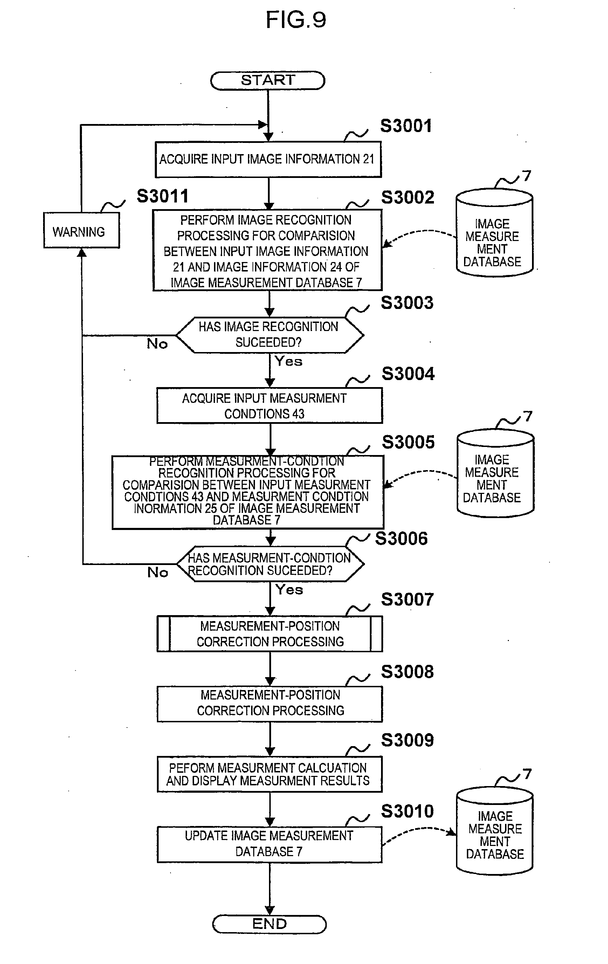

Next, a third embodiment will be described with reference to FIGS. 8 and 9.
In the first embodiment, the one-to-one image recognition calculation is performed for comparison between the input image information 21 and the image information 24 of the image measurement database 7. In the third embodiment, measurement condition recognition calculation is also performed for comparison between the input measurement conditions 43 and the measurement condition information 25 of the image measurement database 7.

(4-1. Image measurement database 7)

FIG. 8 is a diagram showing an example of the image measurement database 7.
The image measurement database 7 of the third embodiment is identical with the image measurement database 7 of the first embodiment. In the third embodiment, recognition calculation is performed for comparison between the input image information 21 and the input measurement conditions 43 and each record of the image measurement information 22.

(4-2. Operation of the measurement-position setting processing section 6)

FIG. 9 is a flowchart showing operation of the measurement-processing setting processing section 6.
The processing in S3001 to S3003 is identical with the processing in S1001 to S1003 of FIG. 3.
When the image recognition has succeeded (Yes in S1003), the ultrasonic diagnostic apparatus 100 acquires the input measurement conditions 43 and stores them in the storage section 3 (S3004). The measurement conditions are various conditions associated with measurement. Examples of the measurement conditions include not only initially set conditions and measurement items, but also measurement timing, time-series frame numbers, the age and sex of a subject, diagnostic information regarding the subject, and the category of the to-be-measured object or the subject. The input measurement conditions 43 may be those input by the operator by use of the operation section 4 and the measurement-item setting section 5.

The measurement condition selection section 13 performs the measurement condition recognition calculation for comparison between the input measurement conditions 43 and the measurement condition information 25 of the image measurement database 7 (S3005). Specifically, priorities are imparted to the above-described items; i.e., initially set conditions, measurement items, measurement timing, time-series frame numbers, the age and sex of a subject, diagnostic information regarding the subject, and the category of the to-be-measured object or the subject. The measurement condition selection section 13 performs the comparison in the order of priority. When the measurement condition information 25 of the image measurement database 7 does not coincide with the input measurement conditions 43 in terms of a certain item, the measurement condition selection section 13 stops the measurement condition recognition calculation. The measurement condition selection section 13 then determines whether or not the measurement condition recognition calculation has succeeded (S3006).
When the measurement condition recognition calculation has succeeded (Yes in S3006), the measurement-position setting section 9 selects a record of the image measurement information 22 which coincides with the input image information 21 in the largest number of items; i.e., a record of the image measurement information 22 which includes the image information 24 most similar to the input image information 21 and the measurement condition information 25 most similar to the input measurement conditions 43. The measurement-position setting section 9 extracts the measurement position information 23 from the record of the image measurement information 22 and sets the measurement position information 23.

The processing in S3007 to S3010 and S3011 is identical with the processing in S1004 to S1007 and S1011 of FIG. 3.

Through the above-described process, the measurement-position setting processing section 6 performs the image recognition calculation and the measurement condition recognition calculation on the basis of the image measurement information 22 of the image measurement database 7 while using the input image information 21 and the input measurement conditions 43 as keys. The measurement-position setting processing section 6 also searches from the image measurement database 7 the image measurement information 22 which includes the image information 24 most similar to the input image information 21 and the measurement condition information 25 most similar to the input measurement conditions 43. The measurement-position setting processing section 6 sets the measurement position for the input image information 21 on the basis of the past measurement position information 23 contained in the searched record of the image measurement information 22.

As described above, the ultrasonic diagnostic apparatus of the third embodiment can search past image measurement information on the basis of not only the input image information but also the input measurement conditions, and automatically set a measurement position. Therefore, the ultrasonic diagnostic apparatus can set an accurate measurement position in accordance with not only the input image information but also the input measurement conditions.

(5. Fourth embodiment: measurement position correction)

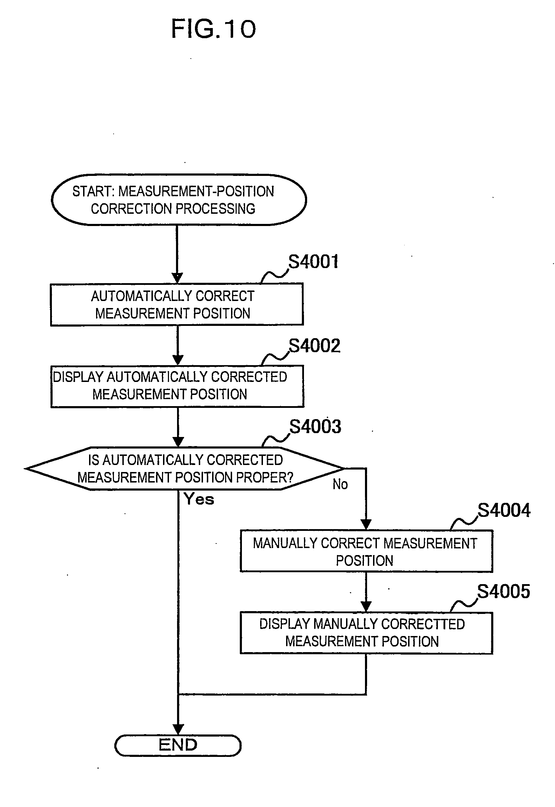

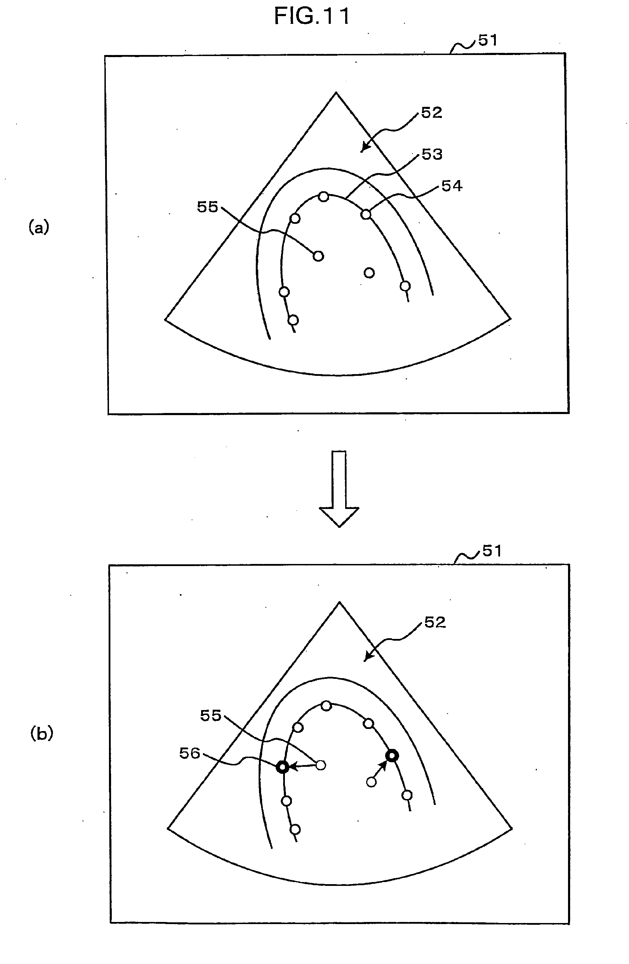

Next, a fourth embodiment will be described with reference to FIGS. 10 and 11. The fourth embodiment relates to the measurement position correction (S1004 of FIG. 3, S2006 of FIG. 7, and S3007 of FIG. 9).

In the first through third embodiments, the measurement position can be accurately set if the image information 24 similar to the input image information 21 exists within the database 7. However, when the image information 24 similar to the input image information 21 does not exist within the database 7, the measurement position cannot be accurately set in some cases. Accordingly, performing measurement-position correction processing is desired.

(5-1. Operation of the measurement-position correction section 10)

FIG. 10 is a flowchart showing operation of the measurement-position correction section 10.

When a measurement position is determined upon completion of the image recognition calculation or the measurement condition recognition calculation, the measurement-position correction section 10 evaluates the measurement position; i.e., determines whether or not the measurement position is proper. When the measurement position is not proper, the measurement-position correction section 10 automatically corrects the measurement position by use of an image feature quantity (S4001). The measurement-position correction section 10 displays the automatically corrected measurement position on the display section 12 (S4002). Notably, the measurement position before the automatic correction may be additionally displayed on the display section 12.

When the operator observes the automatically corrected measurement position and determines that it is not proper (No in S4003), the operator manually corrects the measurement position (S4004). The measurement-position correction section 10 displays the manually corrected measurement position on the display section 12 (S4005).

(5-2. Correction of the measurement position on the basis of edge intensity)

FIG. 11 is a pair of diagrams showing examples of a display screen 51 in measurement-position correction processing. FIG. 11(a) is a diagram showing a state before correction of the measurement position. FIG. 11(b) is a diagram showing a state after correction of the measurement position.

On the display screen 51 of FIG. 11(a), an image 52 and measurement points 54 and 55 before correction are displayed in a superimposed manner. The measurement point 54 is generally set on the endocardial contour 53. However, the measurement point 55 is displayed at a position away from the endocardial contour 53.

On the display screen 51 of FIG. 11(b), the image 52 and the corrected measurement point 56 are displayed in a superimposed manner. The corrected measurement point 56 is a measurement point obtained by correcting the measurement point 55 through the measurement position correction. The corrected measurement point 56 is displayed on the endocardial contour 53.

After arrangement of the measurement points, the measurement-position correction section 10 performs measurement position evaluation for the all the measurement points; i.e., determines whether or not the all the measurement points are proper. In the case shown in FIG. 11, the measurement points are placed on the endocardial contour 53. Therefore, for the measurement position evaluation, an edge intensity is desirably calculated as the image feature quantity.

When the edge intensity is high, the measurement position is proper, and when the edge intensity is low, the measurement position is not proper. The measurement-position correction section 10 corrects the measurement point 55 to the measurement point 56 such that the edge intensity increases. For the measurement position correction, the edge intensity in the vicinity of the measurement point 55 is calculated, and a position where the intensity becomes the maximum is searched, whereby the measurement point 56 can be calculated.

Notably, the image feature quantity is desirably changed in accordance with the positions where the measurement points are arranged or an object on which the measurement points are arranged. Further, when the automatically corrected measurement position is not proper, the operator can manually correct the measurement position by operating the input devices such as a mouse.

(5-3. Correction of measurement position on the basis of image information in the vicinity of the measurement position)

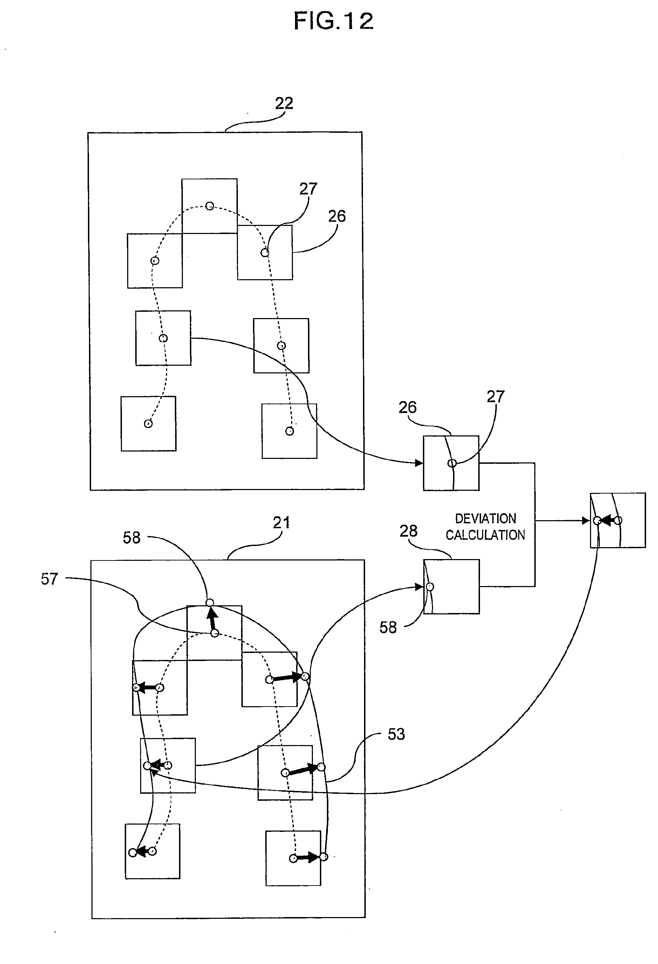

As shown in FIG. 12, the measurement-position correction section 10 uses the measurement position information 23 within the corresponding record of the image measurement database 7 and the image information 26 of a region in the vicinity of the measurement position indicated by the measurement position information 23.

For example, in the case where a measurement position is set on the surface of the endocardial contour 53, the corresponding image measurement information 22 held in the image measurement database 7 is referred to. The measurement position information 23 contained in the image measurement information 22 shows measurement points 27.

When the image measurement information 22 and the input image information 21 are compared with each other, in some cases, a discrepancy arises between image information 26 of a region in the vicinity of a measurement point 27 and image information 28 of a region in the vicinity of a corresponding measurement point 57. Notably, the coordinates of the measurement point 57 of the input image information 21 are set to be identical with those of the measurement point 27 of the image measurement information 22. Further, the shapes of the region (the image information 26) in the vicinity of the measurement point 27 and the region (the image information 28) in the vicinity of the measurement point 57 are rectangular. However, no limitation is imposed on the shapes.

In order to shift each measurement point 57 initially set on the input image information 21 to a corresponding measurement point 58 which is the correct point, the deviation of the measurement point 58 from the measurement point 57 is calculated. For example, image correlation processing such as a block matching method can be used for calculation of the deviation. The image correlation processing or the like is performed so as to compare the image information 26 of the region in the vicinity of the measurement point 27 and the image information 28 of the region in the vicinity of the measurement point 57, to thereby obtain the deviation. Subsequently, the initially set measurement point 57 is shifted by the deviation to the correct measurement point 58.

Alternatively, the measurement point 57 may be corrected as follows. The similarity between the image information 26 of the region in the vicinity of the measurement point 27 and the image information 28 of the region in the vicinity of the measurement point 57 is calculated, and the measurement point 57 is corrected by use of an active contour model (Non-patent Document: M. Kass et al. Snakes: Active Contour Models, International Journal of Computer Vision, 321 - 331, 1988) in which the similarity is used as an image energy. The following Equation (1) represents the entire energy E. In Equation (1), v(s) represents a measurement point, the above-described similarity and/or edge intensity is set to the image energy $E_{image}$, and a feature quantity regarding the contour shape such as a curvature is set to the internal energy $E_{int}$. The measurement point can be fitted to a smooth surface like a wall surface by minimizing the energy E. Thus, accuracy of measurement position setting is improved, and setting in consideration of the smoothness of the organ tissue becomes possible.

[Equation 1]

$$E = \int E_{\text{int}}(v(s)) + E_{image}(v(s))ds \quad (1)$$

Further, another example in which dynamic image processing is used is contour correction processing performed by use of an active shape model or an active appearance model.

As described above, in the fourth embodiment, measurement-position correction processing is performed. Therefore, when the measurement position set on the basis of the past image measurement information is not proper, the measurement position can be corrected on the basis of the input image information. Further, when the corrected measurement position is not proper, the operator can manually correct the measurement position.

(6. Fifth embodiment: setting of a region of interest)

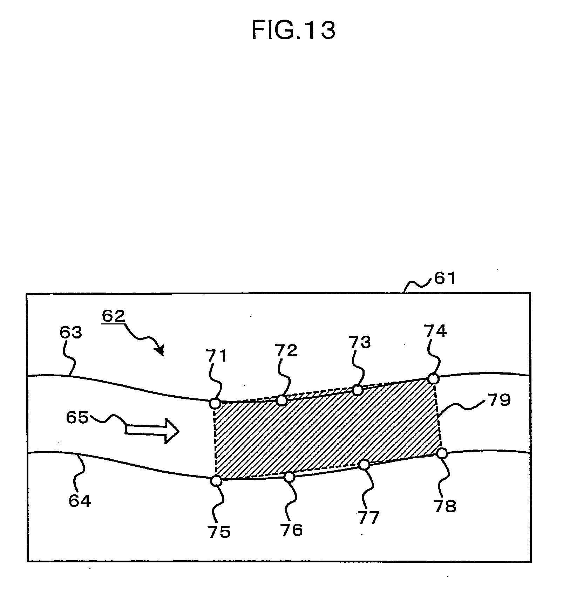

Next, a fifth embodiment will be described with reference to FIG. 13.
In the first through fourth embodiments, measurement points are set and displayed as a measurement position. In the fifth embodiment, a region of interest is set and displayed on the basis of measurement points.
FIG. 13 is a diagram showing setting of a region of interest 79 in an ultrasonic image 61.
In the ultrasonic image 61, a carotid artery 62 is displayed. The carotid artery 62 is a blood vessel in which blood flows in a direction of arrow 65. Wall surfaces 63 and 64 of the carotid artery 62 are portions whose edges are clearly extracted on the ultrasonic image 61.
In the case where the past image information and measurement point information of the carotid artery are recorded in the image measurement database 7, measurement points can be arranged on an ultrasonic image of the carotid artery through processing similar to that employed in the first through fourth embodiments. Since a specific procedure for placing measurement points is described in the first through fourth embodiments, its description will not be repeated.
The measurement-position setting section 9 sets measurement points 71 to 78 on the ultrasonic image 61 of the carotid artery 62 on the basis of the past image information and measurement point information of the carotid artery held in the image measurement database 7.
The measurement-position setting processing section 6 sets a region of interest 79 by connecting the measurement points 71, 74, 78, and 75 at the corners. The frame of the region of interest 79 is displayed on the display section 12 by, for example, a broken line. The measurement calculation section 11 performs a predetermined measurement calculation for the set region of interest 79, and displays the measurement results on the display section 12. For example, the measurement calculation section 11 performs Doppler calculation for the region of interest 79, and performs CFM (color flow mapping) to thereby display the blood flow image in color.
Notably, in FIG. 13, the measurement-position setting processing section 6 sets the region of interest 79 by connecting the measurement points 71, 74, 78, and 75 at the corners. However, the method of setting the region of interest is not limited thereto. The region of interest may be set by connecting all the measurement points 71 to 78. Further, the region of interest may be partially set by connecting the measurement points 72, 73, 76, and 77 only, which are measurement points other than those at the corners.
As described above, in the fifth embodiment, once the ultrasonic probe 1 is brought into contact with a subject to thereby obtain an ultrasonic signal and display an ultrasonic image, a region of interest 79 is automatically set, and, for example, a blood flow image is displayed in the set region of interest 79. Therefore, the operator is not required to operate the trackball or the like of the operation section 4. As a result, since operation of the trackball or the like of the operation section 4 becomes unnecessary, it becomes possible to shorten a time required for diagnosis using the ultrasonic image diagnostic apparatus. Further, since a region of interest is accurately set along the blood vessel, unnecessary CFM processing, such as that for regions outside the blood vessel, is not performed. Therefore, the ultrasonic image can be displayed without greatly decreasing the frame rate.

(7. Sixth embodiment: measurement of tissue dynamics)

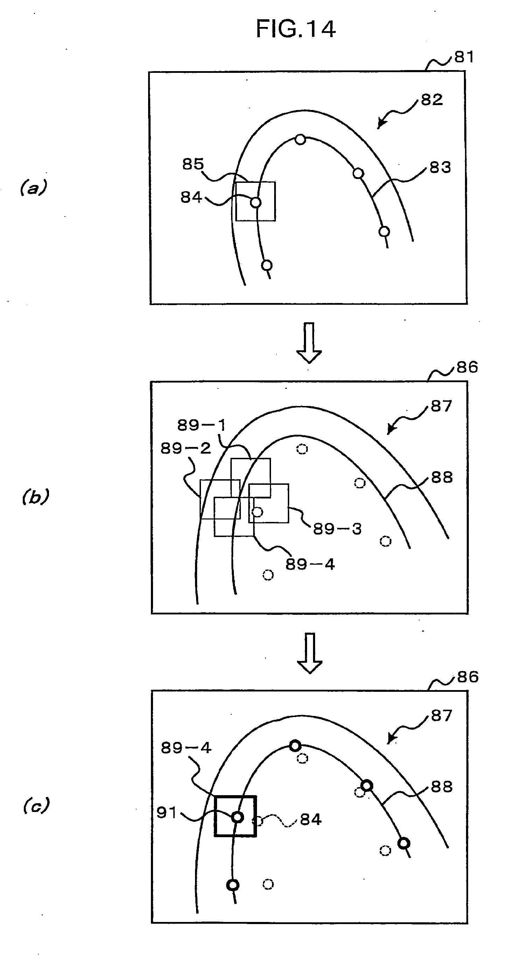

Next, a sixth embodiment will be described with reference to FIG. 14. The sixth embodiment relates to measurement of the tissue dynamics, which is movement of each tissue such as the cardiac muscle.
FIG. 14 is a set of diagrams showing tracking of measurement points in an ultrasonic image.
In the case where the past image information and measurement point information of the cardiac muscle are recorded in the image measurement database 7, measurement points can be arranged on an ultrasonic image of the cardiac muscle through processing similar to that employed in the first through fourth embodiments. Since a specific procedure for placing measurement points is described in the first through fourth embodiments, its description will not be repeated.
As shown in FIG. 14(a), the measurement-position setting section 9 reads from the storage section 3 a frame image captured at a predetermined point in time, and displays an ultrasonic image 81 on the display section 12. The ultrasonic image 81 includes an image of the cardiac muscle 82.
The measurement-position setting section 9 sets measurement points 84 on the ultrasonic image 81 of the cardiac muscle 82 on the basis of the past image information and measurement point information of the cardiac muscle held in the image measurement database 7. The measurement points 84 are arranged along the endocardial contour 83 of the cardiac muscle 82.
The tracking section 14 sets a cutout image 85 of a predetermined region containing each measurement point 84.
As shown in FIG. 14(b), the tracking section 14 reads the next frame image from the storage section 3, and displays an ultrasonic image 86 on the display section 12. The ultrasonic image 86 includes an image of the cardiac muscle 87. The cardiac muscle 82 is displayed as the cardiac muscle 87 because of movement of the tissue. The endocardial contour 83 of the cardiac muscle 82 has expanded, and is displayed as the endocardial contour 88 of the cardiac muscle 87.

The tracking section 14 successively extracts local images 89-1, 89-2, etc., which have the same size as the cutout image 85, from the ultrasonic image 86. The tracking section 14 calculates the degree of coincidence between the cutout image 85 and the local images 89-1, 89-2, etc. For example, image correlation processing such as a block matching method can be used for calculation of the degree of coincidence between images.

As shown in FIG. 14(c), the tracking section 14 selects the position of the local image 89 (in FIG. 14(c), the local image 89-4) whose degree of coincidence is the greatest. The tracking section 14 calculates the position of the selected local image 89 as a measurement point 91 after the movement. The tracking section 14 displays the measurement point 91 such that it is superimposed on the cardiac muscle 87 of the ultrasonic image 86. The measurement point 91 is arranged along the endocardial contour 88, which corresponds to the expanded endocardial contour 83.

As described above, the ultrasonic diagnostic apparatus of the sixth embodiment sets measurement points for a frame image captured at a predetermined point in time on the basis of the past image information and measurement position information, and flows the measurement points for the next and subsequent frame images on the basis of the degree of coincidence between the images. Accordingly, the ultrasonic diagnostic apparatus can automatically set the measurement points, and move the measurement points in accordance with movement of the tissue, to thereby accurately measure the tissue dynamics.

Notably, in place of the tracking of the measurement points by the tracking section 14, the same processing as that of the first through fourth embodiments, etc. may be performed for each of frame images captured at different points in time. That is, the measurement-position setting processing section 6 may be configured to set the measurement points for each frame on the basis of the past image information of the cardiac muscle and the past measurement position information.

(8. Others)

The above-described embodiments can be applied to images displayed real-time, images accumulated in a cine memory, images stored in a storage apparatus in a moving-picture format (e.g., the AVI format).

The measurement position setting can be performed one time or a plurality of times in each heart beat in synchronism with a certain time phase(s) of an ECG (Electrocardiogram), when a freeze button of the operation section is pressed so as to temporarily stop image capturing, when a user selects a frame from a group of frames held in a cine memory after the freeze, when the user selects a frame from a group of frames held in a motion picture file within the storage apparatus, or the like timing. In this case, the measurement position information is desirably held in a database which includes image information corresponding to the above-described timing.

Further, classification to long-axis tomogram, short-axis tomogram, four-chamber tomogram, and two-chamber tomogram is possible. Therefore, in the case where the ultrasonic diagnostic apparatus is applied to stress echo, the ultrasonic diagnostic apparatus automatically determines the view type for the operator, to thereby mitigate the burden of the user. Further, in the case where the positions of myocardial segments (e.g., segments recommended by ASE (American Society of Electrocardiography) are held in the database and these positions are displayed as set positions, the positions of the myocardial segments can be grasped at a glance, and the operation by the operator can be assisted. Accordingly, the operator is only required to operate the ultrasonic probe and give a point number. Therefore, complexity of image classification is mitigated, and the degree of freedom in the order of operations (such as the order of acquiring images) increases.

In the contrast medium mode, changes in brightness of a certain tissue with time are analyzed by means of TIC (Time Intensity Curve). At that time, an operator must set a frame of a certain time which the operator wants to measure and a position of a subject tissue for each frame by operating the input devices such as the trackball. If a database for the case where a contrast medium is used is held, the positions for measurement of brightness are automatically set, whereby the burden imposed on the operator is mitigated.

If a window for Doppler measurement and a measurement region for strain measurement are held in the database, automation of these measurements is similarly possible.

(9. Seventh embodiment)

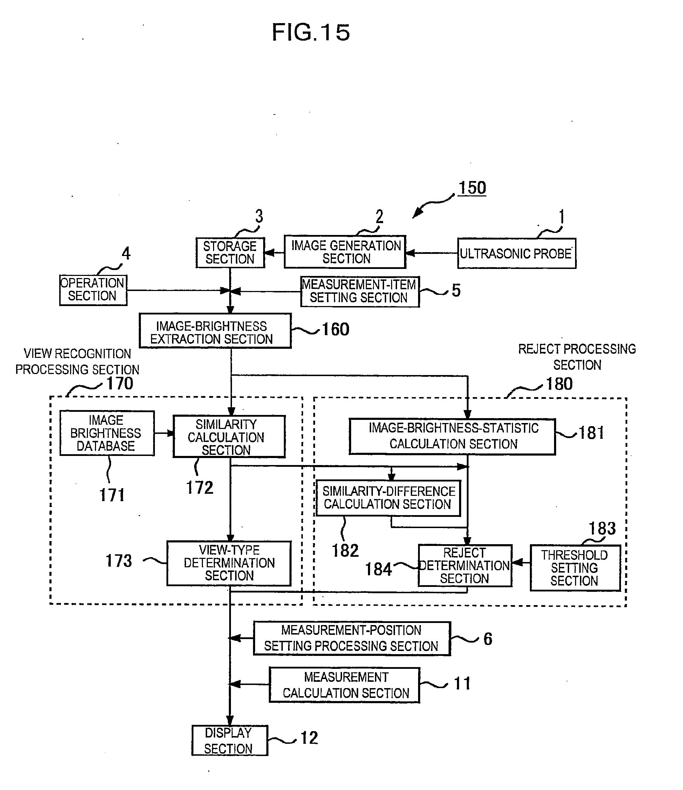

Next, a seventh embodiment will be described with reference to FIGS. 15 to 24.

(9-1. Configuration of an ultrasonic diagnostic apparatus)

The configuration of an ultrasonic diagnostic apparatus 150 will be described with reference to FIG. 15.
FIG. 15 is a diagram showing the configuration of the ultrasonic diagnostic apparatus 150.
The ultrasonic diagnostic apparatus 150 comprises an ultrasonic probe 1 for transmitting and receiving ultrasonic waves; an image generation section 2 for generating an image from an ultrasonic signal; a storage section 3 which serves as a storage area for storing the image; an operation section 4 for enabling an operator to operate the apparatus by use of input devices; a measurement-item setting section 5 for setting measurement items; an image-brightness extraction section 160 for extracting brightness values from an image; a view recognition processing section 170; a reject processing section 180; a measurement-position setting processing section 6 for setting a measurement position; a measurement calculation section 11 for performing measurement calculation on the basis of the set measurement position; and a display section 12 for displaying the measurement position and measurement results.

Since the ultrasonic probe 1, the image generation section 2, the storage section 3, the operation section 4, the measurement-item setting section 5, the measurement-position setting processing section 6, the measurement calculation section 11, and the display section 12 of FIG. 15 are identical with those of FIG. 1, their descriptions will not be repeated.

The image-brightness extraction section 160 extracts brightness values of a region of an image in which the heart is depicted.

The view recognition processing section 170 recognizes the view type of an input image by making use of a pattern of the brightness values obtained in the image-brightness extraction section 160.

The input image is an image selected from images acquired by the ultrasonic probe 1 and the image generation section 2. Notably, an image stored in the storage section 3 may be used as the input image.

The view recognition processing section 170 includes an image brightness database 171, a similarity calculation section 172, and a view-type determination section 173.

The image brightness database 171 stores the view types and brightness values of standard cross-sectional images in a database format. Notably, the image brightness database 171 will be described later.

The similarity calculation section 172 calculates the similarity between the brightness values obtained in the image-brightness extraction section 160 and the brightness values of the standard cross-sectional image of each view type within the image brightness database 171. The result of calculation of the similarity is displayed in scalar or vector.

The view-type determination section 173 compares the similarities of the images of the respective view types calculated by the similarity calculation section 172, and determines the view type of the image having the greatest similarity.

The reject processing section 180 rejects the input image on the basis of the similarity obtained in the similarity calculation section 172 or the like. The reject processing section 180 includes an image-brightness-statistic calculation section 181, a similarity-difference calculation section 182, a threshold setting section 183, and a reject determination section 184.

The image-brightness-statistic calculation section 181 calculates a statistic of the brightness values obtained in the image-brightness extraction section 160. The statistic may be a standard statistic (e.g., mean or variance) or a second-order texture statistic.

The similarity-difference calculation section 182 calculates a similarity difference on the basis of similarities calculated in the similarity calculation section 172. The similarity difference is the difference between the similarity of a standard cross-sectional image which has the greatest similarity, and the similarity of a standard cross-sectional image which has the second greatest similarity. The determination as to whether the view type of the input image is vague is made by use of the similarity difference.

The threshold setting section 183 sets a threshold value for performing reject processing. The threshold setting section 183 sets a threshold value for each of the image brightness statistic obtained in the image-brightness-statistic calculation section 181, the similarity obtained in the similarity calculation section 172, and the similarity difference obtained in the similarity-difference calculation section 182. Notably, respective default values may be set as the threshold values. Alternatively, the operator may individually set the threshold values via the input devices.

The reject determination section 184 performs threshold processing for the image brightness statistic obtained in the image-brightness-statistic calculation section 181, the similarity obtained in the similarity calculation section 172, and the similarity difference obtained in the similarity-difference calculation section 182, while using the threshold values set in the threshold setting section 183, and determines whether or not the input image is to be rejected.

Notably, the display section 12 displays, for example, the result of the determination by the view-type determination section 173, the result of the determination by the reject determination section 184, the name of the view type, the similarity, a graph showing the similarity, and a warning indicating that the input image has been rejected and an input image must be acquired again.

When the view type displayed on the display 12 is incorrect, the operator can manually set a different view type via the operation section 4.

(9-2. Image brightness database 171)

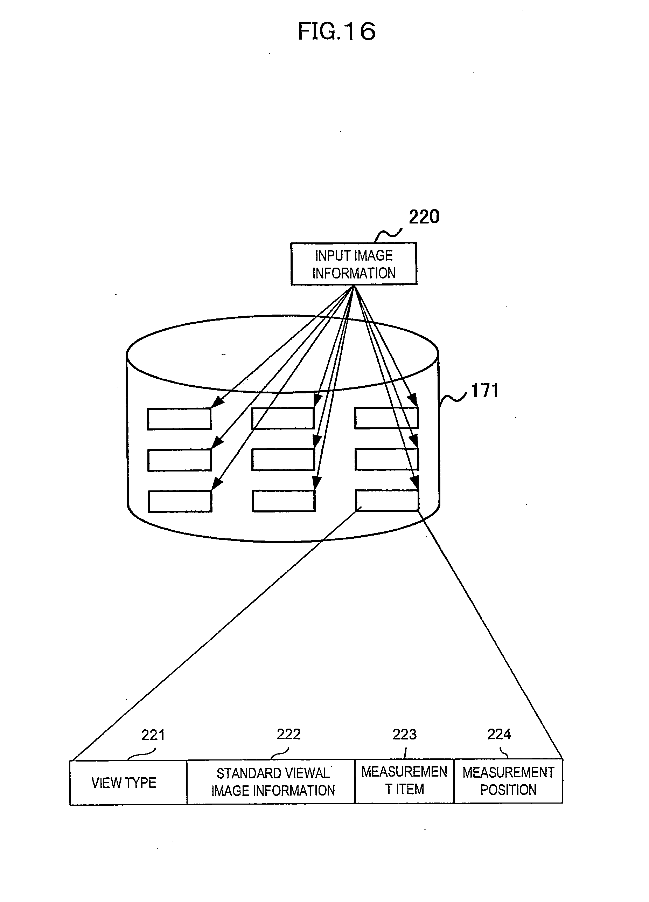

FIG. 16 is a diagram showing an example of a record held in an image brightness database 171.

The image brightness database 171 holds information regarding a standard cross-sectional image for each view type. The information regarding the standard cross-sectional image may be brightness values or image data having undergone predetermined processing. For example, the predetermined processing may be feature extraction processing based on pattern recognition processing such as a subspace method, or compression coding processing. Notably, images obtained in the past may be held in the image brightness database 171 in a database format.

The image brightness database 171 shown in FIG. 16 holds a view type 221, standard cross-sectional image information 222, a measurement item 223, and a measurement position 224 such that they are related to one another. The view type 221 represents the type of the image. For example, the view type 221 is "long-axis," "short-axis," "two-chamber," or "four-chamber." The standard cross-sectional image information 222 is information (brightness values, etc.) regarding a standard cross-sectional image belonging to the view type 221. The measurement item 223 shows a measurement item corresponding to the view type 221. The measurement position 224 is information regarding the measurement position. For example, the measurement position 224 is a set of coordinate values representing measurement points, a measurement region, or a group of contour points defining a measurement region.

(9-3. Reject processing based on presence/absence of an image of the heart)

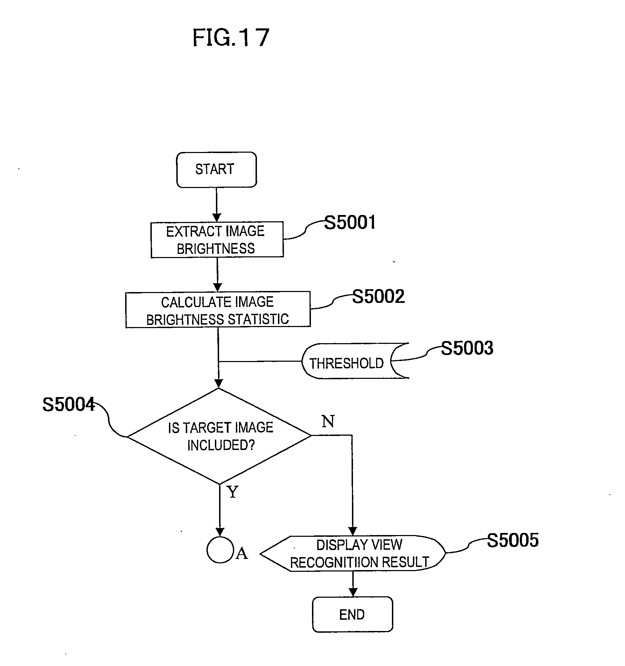

FIG. 17 is a flowchart showing reject processing based on an image-brightness statistic of an input image. The ultrasonic diagnostic apparatus 150 acquires an input image 220, which is image data of a subject, and holds it in the storage section 3. The input image 220 may be image data generated by the image generation section 2 through use of the ultrasonic probe 1, image data generated by a different medical image diagnostic apparatus, or image data which was acquired in the past and stored in the hard disk or the like of the storage section 3.

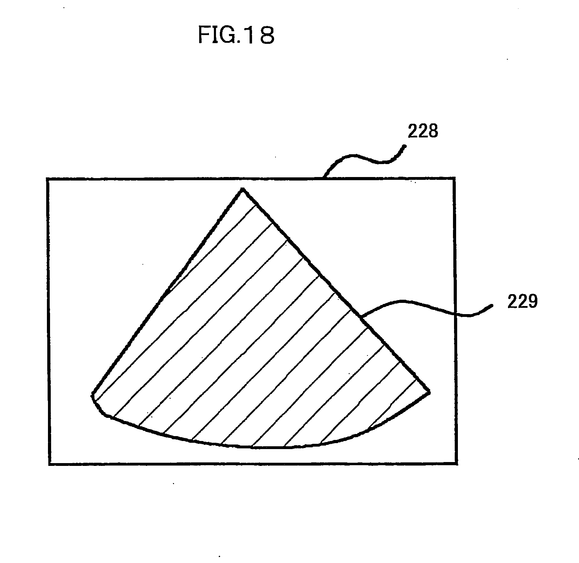

FIG. 18 is a diagram showing a screen 228 and an ultrasonic image 229 displayed on the display section 12. The image-brightness extraction section 160 extracts brightness values of the input image 220 (S5001). The image-brightness extraction section 160 may be configured to extract brightness values of the ultrasonic image 229 of FIG. 18 within the entire view angle of the probe, or brightness values of a portion of the ultrasonic image 229.
The image-brightness-statistic calculation section 181 calculates a statistic of the brightness values of the input image 220 extracted through the processing in S5001 (S5002). The statistic may be a standard statistic (e.g., mean, variance, strain, kurtosis) or a second-order texture statistic (e.g., the feature quantity of a gray-level co-occurrence matrix).

The ultrasonic image of the heart is an image in which probability distributions of brightness of the cardiac cavities, the cardiac muscle, and other regions are mixed. The image brightness statistic of the ultrasonic image of the heart exhibits a characteristic value. When the prove 1 is not in contact with the body surface of a subject and the input image 220 does not contain an image of the heart, a statistic peculiar to the ultrasonic image cannot be obtained, and a statistic peculiar to noise is obtained.

The threshold setting section 183 sets a threshold value for the image brightness statistic of the input image 220 (S5003). The reject determination section 184 performs threshold processing so as to determine whether or not the input image 220 includes an ultrasonic image of the heart (S5004).

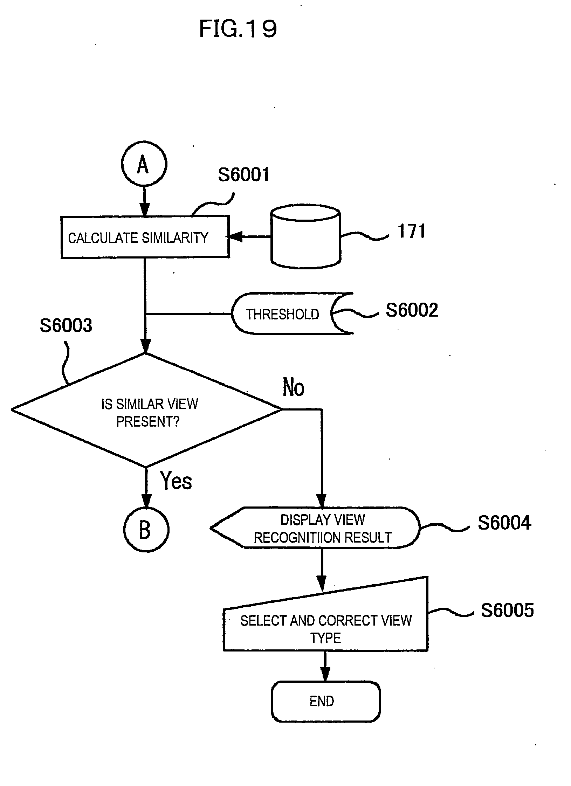

When the reject determination section 184 determines that the input image 220 does not include an ultrasonic image of the heart (No in S5004), the display section 12 displays, as a view recognition result, a message indicating that the input image 220 was rejected, and prompts the operator to acquire the input image 220 again (S5005).
When the reject determination section 184 determines that the input image 220 includes an ultrasonic image of the heart (Yes in S5004), the ultrasonic diagnostic apparatus 150 proceeds to steps shown in FIG. 19.

Through the above-described process, the ultrasonic diagnostic apparatus 150 extracts brightness values of the input image 220, calculates an image brightness statistic of the input image 220, and determines on the basis of the threshold value of the image brightness statistic whether or not the input image 220 contains an ultrasonic image of the heart. When the ultrasonic diagnostic apparatus 150 determines that the input image 220 does not include an ultrasonic image of the heart, the apparatus rejects the input image 220, and prompts the operator to acquire the input image 220 again.

As described above, determination as to whether or not the operator has acquired an ultrasonic image of the heart by properly operating the probe can be made on the basis of a reject criterion, i.e., the result of determination as to whether the statistical characteristic of the input image coincides with the characteristic of the ultrasonic image of the heart. Further, when the input image is rejected at that point in time, the view-type recognition processing becomes unnecessary to be performed for that input image. Accordingly, the processing can be performed quickly and efficiently.

Notably, the operator may input the threshold value by use of the input devices or use a previously set initial value as the threshold value.
Further, a change in the threshold value results in changes in the reject ratio and the false recognition ratio. When the reject ratio is high, the false recognition ratio decreases. However, since the view type of an image which differs from the standard cross-sectional image only slightly cannot be recognized, the operability deteriorates. In contrast, when the reject ratio is low, the view type of an image which slightly differs from the standard cross-sectional image can be recognized. However, the false recognition ratio increases. Therefore, in place of the threshold value, the reject ratio or the false recognition ratio may be used so as to adapt the operation feeling of the operator. In this case, a reject ratio or a false recognition ratio which is estimated from the threshold value may be displayed.

(9-4. Reject processing based on similarity)

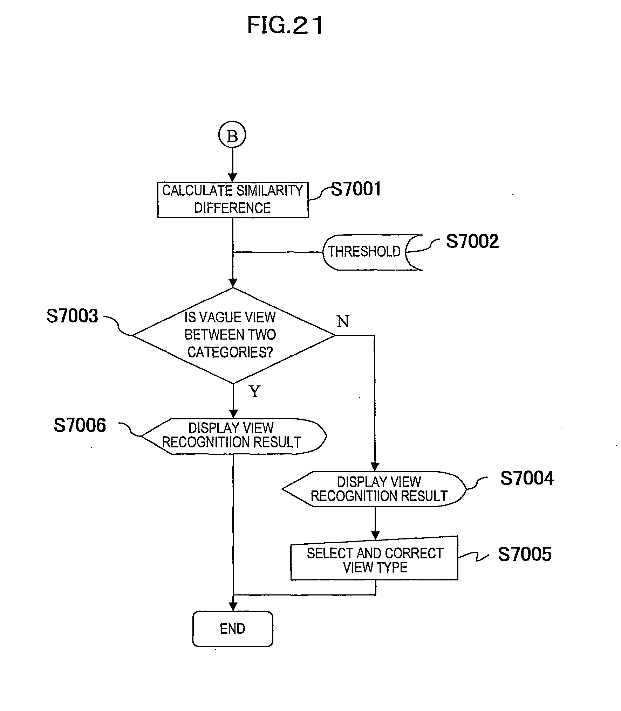

FIG. 19 is a flowchart showing reject processing based on similarity.
When a "Yes" determination is made in S5004 of FIG. 17, the processing in S6001 and subsequent steps of FIG. 19 is executed.
The similarity calculation section 172 calculates the similarity between the input image 220 and the standard cross-sectional image held in the image measurement database 6 for each view type (S6001).
The threshold setting section 183 sets a threshold value for the similarity (S6002). The reject determination section 184 performs threshold processing so as to determine whether or not a standard cross-sectional image similar to the input image 220 is present (S6003).
When the reject determination section 184 determines that a standard cross-sectional image similar to the input image 220 is not present (No in S6003), the display section 12 displays, as a view recognition result, a message indicating that the input image 220 was rejected, and prompts the operator to acquire the input image 220 again (S6004). Notably, when the operator can specify the view type of the input image 220, the operator can manually select and change the view type of the input image 220 via the input devices (S6005).
When the reject determination section 184 determines that a standard cross-sectional image similar to the input image 220 is present (Yes in S6003), the ultrasonic diagnostic apparatus 150 proceeds to steps shown in FIG. 21.

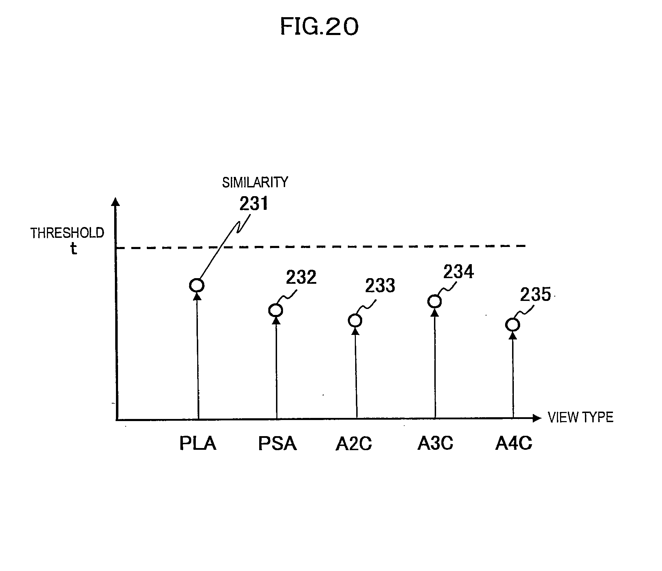

FIG. 20 is a diagram showing the similarity between the input image 220 and a standard cross-sectional image for each view type.
For the following description, it is assumed that the similarity is calculated in scalar, and the degree of similarity increases with the value of the similarity.
FIG. 20 shows points 231 to 235 which represent similarities between the input image 220 and respective standard cross-sectional images of five view types; i.e., PLA (parasternal long-axis view), PSA (parasternal short-axis view), A2C (apical two-chamber view), A3C (apical three-chamber view), and A4C (apical four-chamber view). All the similarities indicated by the points 231 to 235 are smaller than the threshold value "t" set by the threshold setting section 183. Therefore, a standard cross-sectional image similar to the input image 220 is not present in the standard cross-sectional images of the above-described five view types, and the reject determination section 184 determines that the input image 220 is to be rejected (No in S6003).
Through the above-described process, the ultrasonic diagnostic apparatus 150 calculates the similarity between the input image 220 and the standard cross-sectional image held in the image measurement database 6 for each view type, and determines on the basis of the threshold value of the similarity whether or not a standard cross-sectional image similar to the input image 220 is present. When the ultrasonic diagnostic apparatus 150 determines that a standard cross-sectional image similar to the input image 220 is not present, the apparatus displays, as a view recognition result, a message indicating that the input image 220 was rejected, and prompts the operator to acquire the input image 220 again.
As described above, when the view type of an acquired ultrasonic image of the heart is not a view type necessary for the measurement (when the input image differs from the standard cross-sectional images of the all view types), the ultrasonic diagnostic apparatus 150 rejects the input image, and prompts the operator to again acquire an input image of a view type necessary for the measurement. Accordingly, the processing can be performed quickly and efficiently. Notably, the setting of the threshold value may be performed by setting a threshold value for the similarity or setting a threshold value for the reject ratio or the false recognition ratio.

(9-5. Reject processing based on similarity difference)

FIG. 21 is a flowchart showing reject processing based on similarity difference.
When a "Yes" determination is made in S6003 of FIG. 19, the processing in S7001 and subsequent steps of FIG. 21 is executed.
The similarity-difference calculation section 182 calculates a similarity difference by making use of the result output from the similarity calculation section 172 (S7001). The similarity difference is the difference between the similarity of a standard cross-sectional image which is most similar to the input image 220 and the similarity of a standard cross-sectional image which is second-most similar to the input image 220. The similarity difference is an indicator representing whether or not the input image 220 resembles two standard cross-sectional images and its view type is vague.
The threshold setting section 183 sets a threshold value for the similarity difference (S7002). The reject determination section 184 performs threshold processing so as to determine whether or not the view type of the input image 220 is vague (S7003).
When the reject determination section 184 determines that the view type of the input image 220 is vague (Yes in S7003), the display section 12 displays, as the view recognition result, a message indicating that the input image 220 was rejected, and prompts the operator to acquire the input image 220 again (S7004). Notably, when the operator can specify the view type of the input image 220, the operator can manually select and change the view type of the input image 220 via the input devices (S7005).

When the reject determination section 184 determines that the view type of the input image 220 is not vague (No in S7003), the display section 12 displays, as the view recognition result, the final view type of the input image 220, and the similarity between the input image and the standard cross-sectional image of each view type, etc. (S7006).

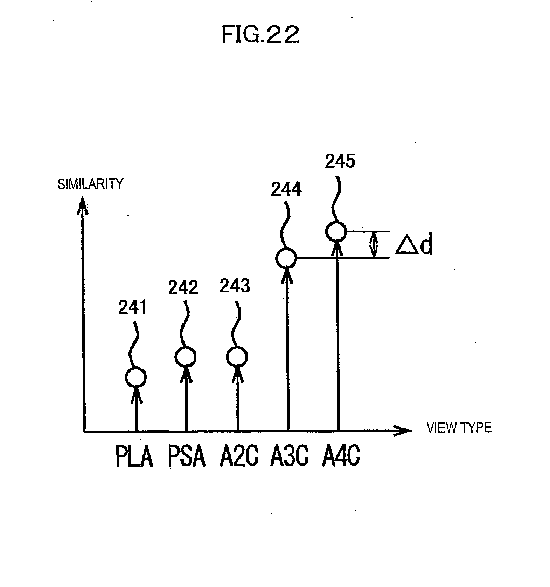

FIG. 22 is a diagram showing the similarity between the input image 220 and a standard cross-sectional image for each view type.

FIG. 22 shows points 241 to 245 which represent similarities between the input image 220 and respective standard cross-sectional images of the five view types; i.e., PLA (parasternal long-axis view), PSA (parasternal short-axis view), A2C (apical two-chamber view), A3C (apical three-chamber view), and A4C (apical four-chamber view). The similarity represented by the point 245 is the greatest, and the similarity represented by the point 244 is the second greatest. The similarity-difference calculation section 182 calculates the similarity difference "Δd" between the points 244 and 245. When the similarity difference "Δd" is less. than the threshold value set by the threshold setting section 183, the reject determination section 184 determines that the view type of the input image 220 is vague, and rejects the input image 220 (Yes in S7003).

Through the above-described process, the ultrasonic diagnostic apparatus 150 calculates the difference between the similarity of a standard cross-sectional image which is most similar to the input image 220 and the similarity of a standard cross-sectional image which is second-most similar to the input image 220, and determines on the basis of the threshold value of the similarity difference whether or not the view type of the input image 220 is vague. When the ultrasonic diagnostic apparatus 150 determines that the view type of the input image 220 is vague, the apparatus displays, as a view recognition result, a message indicating that the input image 220 was rejected, and prompts the operator to acquire the input image 220 again.

As described above, when an input image; i.e., an ultrasonic image of the heart, is a vague image resembling two standard cross-sectional images, the ultrasonic diagnostic apparatus 150 rejects the input image, and prompts the operator to again acquire an input image of a view type necessary for the measurement. Accordingly, the processing can be performed quickly and efficiently.

Notably, the setting of the threshold value may be performed by setting a threshold value for the similarity difference or setting a threshold value for the reject ratio or the false recognition ratio.

(9-6. Screen display)

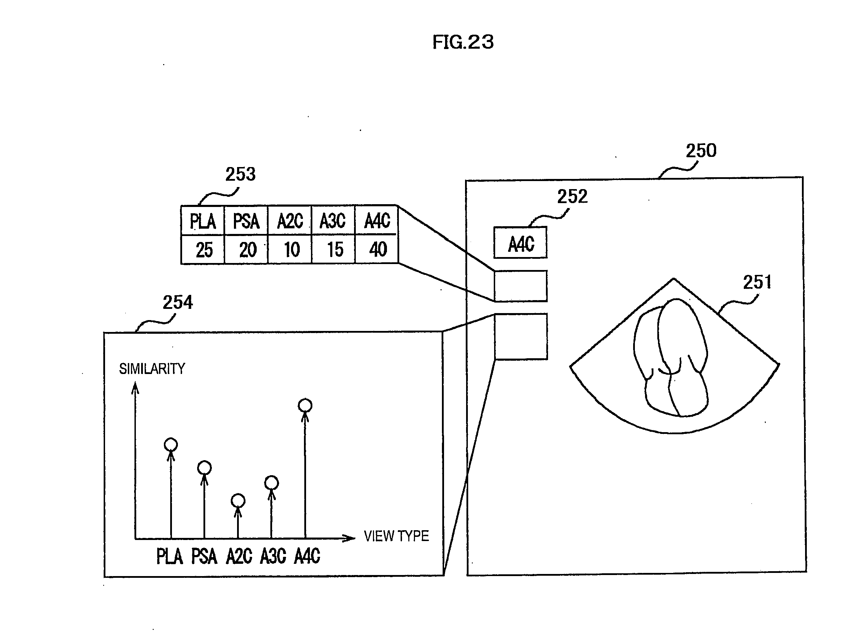

FIG. 23 is a diagram showing an example of a screen 250 displayed by the display section 12.

An ultrasonic image 251 is displayed on the screen 250. In the ultrasonic image 251, an A4C image (apical four-chamber view) is depicted.

The view recognition result is displayed in a view-type display area 252. For example, "4AC" is displayed in the view-type display area 252. Notably, when the input image 220 was rejected, for example, "Rejected" is displayed in the view-type display area 252.

In a similarity display area 253, a similarity is displayed for each view type. In FIG. 23, the similarity of "4AC" is "40" and is the greatest (the greatest similarity).

In a graph display area 254, the contents of the similarity display area 253 are displayed in the form of a graph. This enables the operator to visually grasp the view type of the standard cross-sectional image to which the input image 220 is similar.

No limitation is imposed on the timing at which the view recognition processing is performed. For example, in the case where the view recognition processing is performed every time the R wave appears on an ECG (Electrocardiogram), the view-type display area 252, the similarity display area 253, and the graph display area 254 are updated every time the R wave appears. The operator can adjust the position and angle of the ultrasonic probe 1, while viewing these display areas, so as to depict an image of a view type to be measured.

(9-7. Measurement position setting)

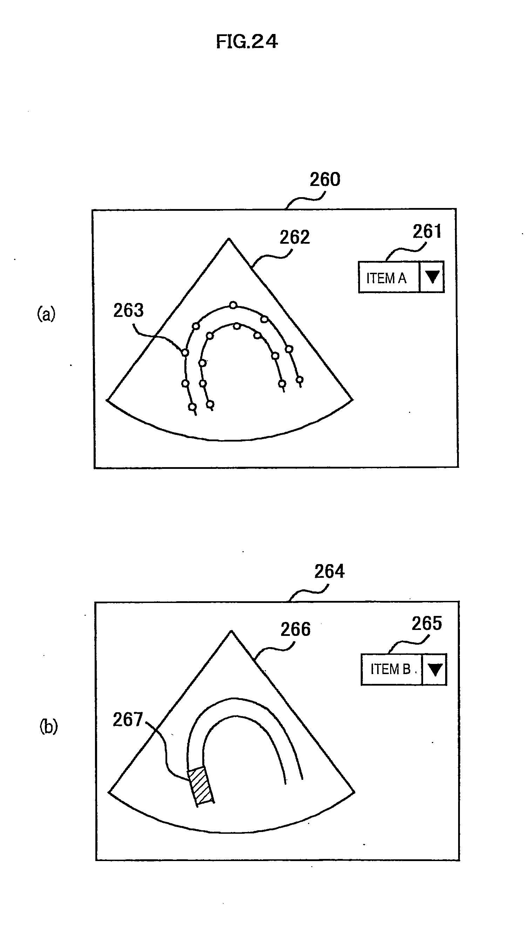

FIG. 24 is a pair of diagrams showing a screen 260 and a screen 264 displayed on the display section 12.

When the operator selects an "item A" on the screen 260 as an measurement item 261 as shown in FIG. 24(a), measurement points 263 corresponding to the measurement item are displayed such that the measurement points 263 are superimposed on an ultrasonic image 262.

When the operator selects an "item B" on the screen 264 as a measurement item 265 as shown in FIG. 24(b), a measurement region 267 corresponding to the measurement item is displayed such that the measurement region 267 is superimposed on an ultrasonic image 266.

Notably, the measurement-item setting section 5 sets the measurement item, and the measurement-position setting processing section 6 sets the measurement points 263 or the measurement region 267 on the basis of the view type 221, the measurement item 223, and the measurement position 224 of the image intensity database 171. The measurement calculation section 11 performs measurement for the set measurement points 263 or measurement region 267, and displays the results of the measurement calculation on the display section 12.

As described above, the ultrasonic diagnostic apparatus 150 can configure a measurement menu corresponding to the view type recognized through the view recognition processing. For example, when the view type of the input image is recognized to be "PSA (parasternal short-axis view)," the ultrasonic diagnostic apparatus 150 limits the measurement menus to those used for "PSA (parasternal short-axis view)," to thereby mitigate the labor of selecting the measurement item.

(9-8. Effects of the seventh embodiment)

As described above, the ultrasonic diagnostic apparatus 150 of the seventh embodiment calculates the image brightness statistic of an input image, the similarities between the input image and the standard cross-sectional images, and the similarity difference, which is the difference between the similarities of the input image and two standard cross-sectional images; sets the threshold value, the reject ratio, or the false recognition ratio for the image brightness statistic, the similarities, and the similarity difference; and then perform the reject processing. Therefore, the ultrasonic diagnostic apparatus 150 can quickly and efficiently acquires an input image of a view type necessary for the measurement.

Conventionally, when input images of a plurality of view types are captured in a stress echo examination, the capturing order must be determined in advance for each view type. In contrast, the ultrasonic diagnostic apparatus 150 of the present invention automatically recognizes and classifies the view type of the input image. Therefore, the capturing order can be freely determined, the labor of setting is mitigated, and the operability is improved.

(10. Eighth embodiment)

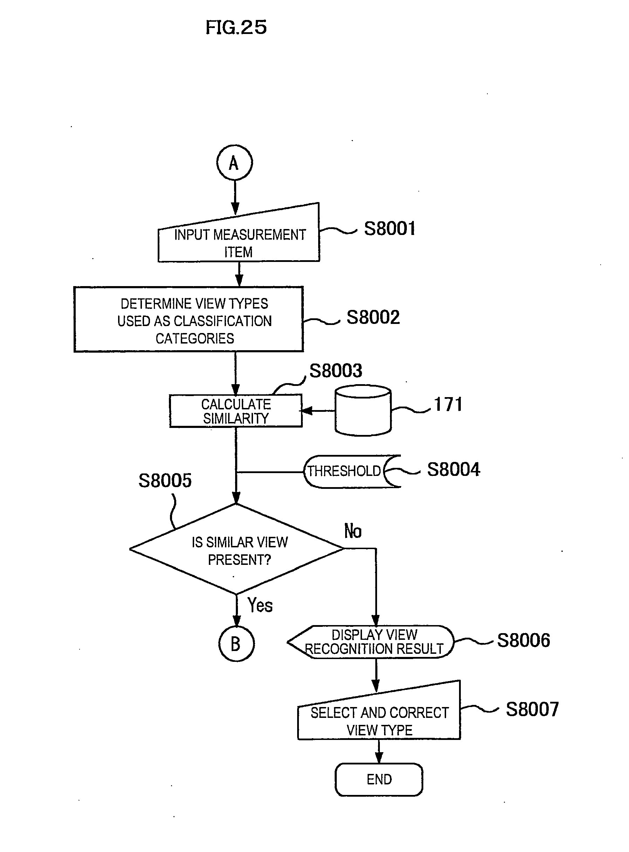

Next, an eighth embodiment will be described with reference to FIGS. 25 to 27.

FIG. 25 is a flowchart showing reject processing based on similarity.

When a "Yes" determination is made in S5004 of FIG. 17, the processing in S8001 and subsequent steps of FIG. 25 is executed.

In the seventh embodiment (FIG. 19), all the view types held in the image brightness database 171 are used as classification categories. In contrast, in the eighth embodiment (FIG. 25), view types which are used as classification categories are determined on the basis of the measurement item.

The ultrasonic diagnostic apparatus 150 accepts the input of a measurement item via the measurement-item setting section 5 (S8001). The ultrasonic diagnostic apparatus 150 extracts view types 221 corresponding to the measurement-item 223 with reference to the image brightness database 171, and uses the extracted view types 221 as classification categories to which the input images are classified (S8002).

The processing in S8003 to S8007 is identical with the processing in S6001 to S6005 of FIG. 19.

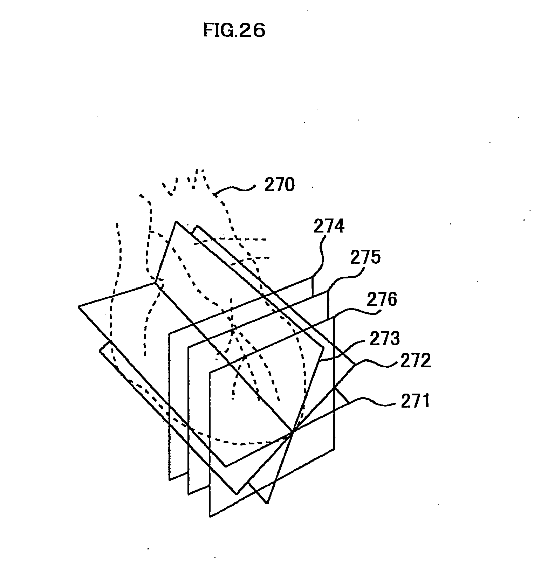

FIG. 26 is a diagram showing view types of the heart.

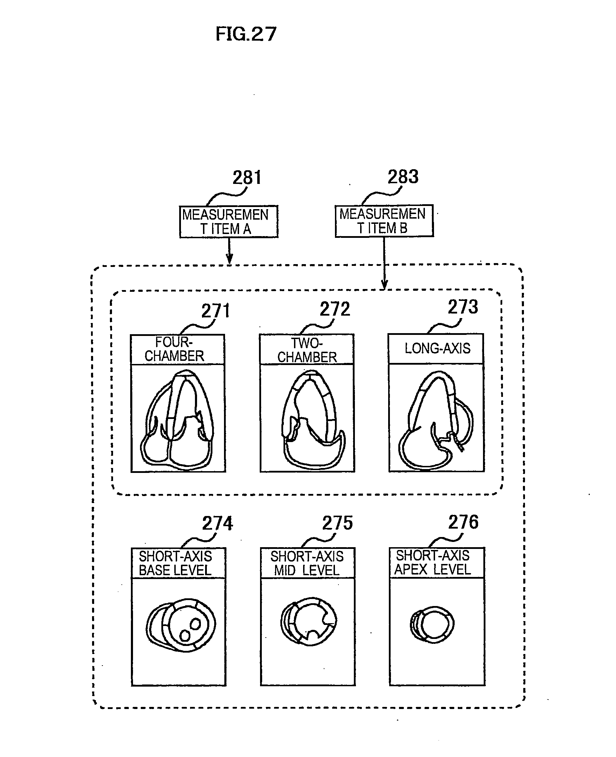

FIG. 27 is a diagram showing measurement items and classification categories.

For the heart 270 of FIG. 26, there are set six standard cross-sectional images; i.e., a standard cross-sectional image 271 (four-chamber), a standard cross-sectional image 272 (two-chamber), a standard cross-sectional image 273 (long-axis), a standard cross-sectional image 274 (short-axis (base level)), a standard cross-sectional image 275 (short-axis (mid level)), and a standard cross-sectional image 276 (short-axis (apex level)).

As shown in FIG. 27, when a measurement item 281 (measurement item A) is set, the ultrasonic diagnostic apparatus 150 selects as classification categories the six view types to which the standard cross-sectional images 271 to 276 belong to; and when a measurement item 283 (measurement item B) is set, the ultrasonic diagnostic apparatus 150 selects as classification categories three view types to which the standard cross-sectional images 271 to 273 belong to.

As described above, in the eighth embodiment, the number of categories to which input images are classified can be changed in accordance with the measurement item. Since the number of categories; for example, the number of measurement menus, can be reduced to a necessary number in accordance with the measurement item. In the above-described example, input images are classified to three categories; i.e., "PLA (parasternal long-axis view)," "PSA (parasternal short-axis view)," and "A3C (apical three-chamber view)." Thus, the recognition ratio can be improved as compared with the case where input images are classified to four or more categories, whereby the operability in the measurement operation can be improved.

(11. Others)

The ultrasonic diagnostic apparatus 150 has been described as performing the reject processing on the basis of the image brightness statistic (FIG. 17), the similarities (FIG. 19), and the similarity difference (FIG. 21) of an input image. However, the reject processing can be performed while the image brightness statistic, the similarities, and the similarity difference are selectively used and combined. Notably, in order to prevent false recognition in the view recognition processing, the reject processing is desirably performed on the basis of all the image brightness statistic (FIG. 17), the similarities (FIG. 19), and the similarity difference (FIG. 21) of an input image.
Further, in the above-described embodiments, the view recognition processing and the reject processing are performed real-time on input images captured by the ultrasonic diagnostic apparatus. However, the embodiments may be modified such that input images captured by the ultrasonic diagnostic apparatus are stored in a motion picture format, and the view recognition processing and the reject processing are performed off-line.

Preferred embodiments of the medical image diagnostic apparatus according to the present invention have been described. However, the present invention is not limited to the above-described embodiments. It is clear that a person with ordinary skill in the art can easily conceive various modifications and changes within the technical idea disclosed herein, and it is contemplated that such modifications and changes naturally fall within the technical scope of the present invention.

**Claims**

1. A medical image diagnostic apparatus comprising image information acquiring means for acquiring image information of a subject; a display section for displaying the image information acquired by the image information acquiring means; and measurement calculation means for performing measurement calculation on the basis of the image information displayed on the display section, the apparatus being **characterized by** further comprising:

   storage means for holding, in a mutually related manner, image information pieces acquired in the past and past measurement position information pieces set for the image information pieces;
   image selection means for recognizing and selecting a past image information piece which is most similar to the input image information; and
   measurement-position setting means for setting a measurement position for the input image information on the basis of the past measurement position information piece corresponding to the past image information piece selected by the image selection means.

2. A medical image diagnostic apparatus according to claim 1, wherein the image selection means performs the image recognition calculation so as to compare the input image information and all the past image information pieces held in the storage means.

3. A medical image diagnostic apparatus according to claim 1, wherein
   the storage means holds the measurement position for each of measurement items;
   the medical image diagnostic apparatus comprises selection means for selecting a measurement item; and
   the measurement-position setting means reads out a measurement position corresponding to the selected measurement item and sets the measurement position.

4. A medical image diagnostic apparatus according to claim 1, wherein
   the storage means holds the past image information pieces classified into a plurality of categories, and representative image information which represents image information pieces belonging to each category;
   the image selection means performs the image recognition calculation so as to compare the input image information and the representative image information held in the storage means to thereby determine the category of the input image information, and performs the image recognition calculation so as to compare the input image information and past image information pieces belonging to the determined category.

5. A medical image diagnostic apparatus according to claim 1, wherein
   the storage means holds past measurement condition information pieces while relating them to the past image information pieces;
   the medical image diagnostic apparatus comprises measurement condition selection means for performing measurement condition recognition calculation so as to compare input measurement condition information and the past measurement condition information pieces held in the storage means and recognizing and selecting a past measurement condition information piece most similar to the input measurement condition information; and the measurement-position setting means sets the measurement position on the basis of a past measurement position information piece corresponding to the past measurement condition information piece selected by the measurement condition selection means.

6. A medical image diagnostic apparatus according to claim 1, comprising:

measurement position evaluation means for determining whether or not the measurement position set by the measurement-position setting means is proper on the basis of an image feature quantity of the input image information; and
measurement-position correction means for correcting the set measurement position on the basis of the image feature quantity of the input image information when the measurement position evaluation means determines that the set measurement position is not proper.

7. A medical image diagnostic apparatus according to claim 1, comprising:

measurement-position correction means for calculating an edge intensity in the vicinity of the measurement position set for the input image information, and correcting the set measurement position to a position where the calculated edge intensity becomes the maximum.

8. A medical image diagnostic apparatus according to claim 1, comprising:

measurement-position correction means for correcting the set measurement position on the basis of a discrepancy between image information in the vicinity of the measurement position set for the input image information and image information in the vicinity of the measurement position indicated by the past measurement position information.

9. A medical image diagnostic apparatus according to claim 1, comprising:

region-of-interest setting means for setting a region of interest on the basis of the measurement position set by the measurement-position setting means.

10. A medical image diagnostic apparatus according to claim 1, comprising:

measurement position tracking means for selecting an image portion of a predetermined range from the input image information, the range including the measurement position set by the measurement-position setting mean, and for tracking a movement of the image portion to thereby move the set measurement position.

11. A medical image diagnostic apparatus according to claim 1, comprising:

image recognition result display means for displaying on the display section a result of the image recognition associated with the input image information when the image recognition by the image selection means has succeeded.

12. A medical image diagnostic apparatus according to claim 1, comprising:

warning processing means for performing warning processing or processing for inputting the input image information again when the image recognition by the image selection means has failed.

13. A medical image diagnostic apparatus according to claim 1, comprising:

measurement position update means for registering the input image information and a measurement position set for the input image information in the storage means for update.

14. A medical image diagnostic apparatus according to claim 1, wherein
the storage means holds brightness information pieces of a plurality of cross-sectional images acquired in the past and view types in a mutually related manner;
the medical image diagnostic apparatus comprises:

similarity calculation means for calculating a similarity between an input image selected from images acquired by the image acquiring means and a plurality of cross-sectional images held in the storage section;

similarity-difference calculation means for calculating a similarity difference which is a difference between the similarity between the input image and a cross-sectional image which is most similar to the input image and the similarity between the input image and another cross-sectional image; and

reject determination means for performing threshold processing for the similarity and the similarity difference so as to determine whether or not the input image is to be rejected.

**15.** A medical image diagnostic apparatus according to claim 14, further comprising:

image-brightness-statistic calculation means for calculating a brightness statistic of the input image, wherein the reject determination means performs the threshold processing for the brightness statistic of the input image so as to determine whether or not the input image is to be rejected.

**16.** A medical image diagnostic apparatus according to claim 14, further comprising:

reject ratio setting means for setting a reject ratio at which the input image is determined to be rejected, wherein the reject determination means performs the threshold processing by use of a threshold value corresponding to the reject ratio set by the reject ratio setting means so as to determine whether or not the input image is to be rejected.

**17.** A medical image diagnostic apparatus according to claim 14, further comprising:

measurement-item setting means for setting a measurement item; and

classification-category determination means for determining view types which are used as classification categories, on the basis of the measurement item set by the measurement-item setting means.

**18.** A medical image diagnostic apparatus according to claim 14, further comprising:

classification means for classifying the input image to a view type of an cross-sectional image which is most similar to the input image.

**19.** A medical image measurement method for displaying image information of a subject and performing measurement calculation on the basis of the displayed image information, the method **characterized by** comprising:

a step of holding, in a storage apparatus, image information pieces acquired in the past and past measurement position information pieces set for the image information pieces such that the image information pieces and the measurement position information pieces are related to each other;

an image selection step of recognizing and selecting a past image information piece which is most similar to the input image information; and

a measurement-position setting step of setting a measurement position for the input image information on the basis of the past measurement position information piece corresponding to the past image information piece selected by the image recognition step.

**20.** A medical image measurement program for causing a computer to acquire image information of a subject, display the acquired image information, and perform measurement calculation on the basis of the displayed image information, the program being **characterized by** causing the computer to perform:

a step of holding, in a storage apparatus, image information pieces acquired in the past and past measurement position information pieces set for the image information pieces such that the image information pieces and the measurement position information pieces are related to each other;

an image selection step of recognizing and selecting a past image information piece which is most similar to the input image information; and

a measurement-position setting step of setting a measurement position for the input image information on the basis of the past measurement position information piece corresponding to the past image information piece selected by the image recognition step.

# FIG.1

100 ULTRASONIC DIAGNOSTIC APPARATUS

```
      3                          2                        1
STORAGE          ◄──  IMAGE GENERATION  ◄──  ULTRASONIC
SECTION                  SECTION                   PROBE
```

```
      4                                   5
OPERATION                      MEASUREMENT-ITEM
 SECTION                       SETTING SECTION
```

6 MEASUREMENT-POSITION SETTING
PROCESSING SECTION

```
         7                           IMAGE SELECTION          8
                                          SECTION
      IMAGE
   MEASUREMENT
    DATABASE                       MEASUREMENT-              9
                                   POSITION SETTING
                                       SECTION
            13
  MEASUREMENT                       MEASUREMENT-             10
   CONDITION                         POSITION
SELECTION SECTION                    CORRECTION
                                      SECTION
```

```
      14                                   11
 TRACKING                          MEASUREMENT
  SECTION                          CALCULATION
                                     SECTION
```

```
                12
             DISPLAY
             SECTION
```

25

# FIG.2

~21

INPUT IMAGE
INFORMATION

~7

**23** | **26** | **24** | **25** | **22** IMAGE
MEASUREMENT
INFORMATION

| MEASUREMENT POSITION INFORMATION | IMAGE INFORMATION AROUND MEASUREMENT POSITION | IMAGE INFORM-ATION | MEASUREMENT CONDITION INFORMATION |
|---|---|---|---|

# FIG.3

START

**~S1001**

ACQUIRE INPUT IMAGE
INFORMATION 21

**~S1011**

WARNING

**~S1002**

PERFORM IMAGE RECOGNITION
PROCESSING FOR COMPARISION
BETWEEN INPUT IMAGE INFORMATION
21 AND IMAGE INFORMATION 24 OF
IMAGE MEASUREMENT DATABASE 7

**~7**

IMAGE
MEASUR-
EMENT
DATABASE

**~S1003**

HAS IMAGE RECOGNITION
SUCEEDED?

No

Yes

**~S1004**

MEASUREMENT-POSITION
CORRECTION PROCESSING

**~S1005**

DISPLAY MEASUREMENT POSITION

**~S1006**

PEFORM MEASURMENT CALCUATION
AND DISPLAY MEASURMENT RESULTS

**~S1007**

UPDATE IMAGE MEASUREMENT
DATABASE 7

**~7**

IMAGE
MEASUR-
EMENT
DATABASE

END

# FIG.4

# FIG.5

(a)

(b)

# FIG.6

# FIG.7

START

↓

S2001
ACQUIRE INPUT IMAGE INFORMATION 21

S2011
WARNING

S2002
PERFORM IMAGE RECOGNITION PROCESSING FOR COMPARISION BETWEEN INPUT IMAGE INFORMATION 21 AND REPRESENTATIVE IMAGE INFORMATION 42 OF CLASSIFIED IMAGE MEASUREMENT DATABASE 41

41
CLASSIFIED IMAGE MEASUREMENT DATABASE

S2003
HAS IMAGE RECOGNITION SUCEEDED?
No / Yes

S2004
PERFORM IMAGE RECOGNITION PROCESSING FOR COMPARISION BETWEEN INPUT IMAGE INFORMATION 21 AND IMAGE INFORMATION 24 OF CLASSIFIED IMAGE MEASUREMENT DATABASE 41

S2005
HAS IMAGE RECOGNITION SUCEEDED?
No / Yes

S2006
MEASUREMENT-POSITION CORRECTION PROCESSING

S2007
DISPLAY MEASUREMENT POSITION

S2008
PEFORM MEASURMENT CALCUATION AND DISPLAY MEASURMENT RESULTS

S2009
UPDATE CLASSIFIED IMAGE MEASUREMENT DATABASE 41

41
CLASSIFIED IMAGE MEASUREMENT DATABASE

↓

END

# FIG.8

INPUT MEASUREMENT CONDTION ~43

INPUT IMAGE INFORMATION ~21

~7

IMAGE MEASUREMENT INFORMATION 22

| 23 | 26 | 24 | 25 |
|---|---|---|---|
| MEASUREMENT POSITION INFORMATION | IMAGE INFORMATION AROUND MEASUREMENT POSITION | IMAGE INFORM-ATION | MEASUREMENT CONDITION INFORMATION |

# FIG.9

START

~ S3001

ACQUIRE INPUT IMAGE INFORMATION 21

~ S3011

WARNING

~ S3002

PERFORM IMAGE RECOGNITION
PROCESSING FOR COMPARISION
BETWEEN INPUT IMAGE INFORMATION
21 AND IMAGE INFORMATION 24 OF
IMAGE MEASUREMENT DATABASE 7

~ 7

IMAGE
MEASURE
MENT
DATABASE

~ S3003

HAS IMAGE RECOGNITION
SUCEEDED?

No          Yes

~ S3004

ACQUIRE INPUT MEASURMENT
CONDTIONS 43

~ S3005

PERFORM MEASURMENT-CONDTION
RECOGNITION PROCESSING FOR
COMPARISION BETWEEN INPUT MEASURMENT
CONDTIONS 43 AND MEASURMENT CONDTION
INORMATION 25 OF IMAGE MEASUREMENT
DATABASE 7

~ 7

IMAGE
MEASURE
MENT
DATABASE

~ S3006

HAS MEASURMENT-CONDTION
RECOGNITION SUCEEDED?

No          Yes

~ S3007

MEASUREMENT-POSITION
CORRECTION PROCESSING

~ S3008

MEASUREMENT-POSITION
CORRECTION PROCESSING

~ S3009

PEFORM MEASURMENT CALCUATION
AND DISPLAY MEASURMENT RESULTS

~ S3010

UPDATE IMAGE MEASUREMENT
DATABASE 7

~ 7

IMAGE
MEASURE
MENT
DATABASE

END

# FIG.10

START: MEASUREMENT-POSITION
CORRECTION PROCESSING

S4001

AUTOMATICALLY CORRECT
MEASUREMENT POSITION

S4002

DISPLAY AUTOMATICALLY CORRECTED
MEASUREMENT POSITION

S4003

IS AUTOMATICALLY CORRECTED
MEASUREMENT POSITION PROPER?

Yes

No

S4004

MANUALLY CORRECT MEASUREMENT
POSITION

S4005

DISPLAY MANUALLY CORRECTTED
MEASUREMENT POSITION

END

## FIG.11

(a)

(b)

# FIG.12

# FIG.13

# FIG.14

# FIG.15

150

3 STORAGE SECTION

2 IMAGE GENERATION SECTION

1 ULTRASONIC PROBE

4 OPERATION SECTION

MEASUREMENT-ITEM SETTING SECTION 5

IMAGE-BRIGHTNESS EXTRACTION SECTION 160

VIEW RECOGNITION PROCESSING SECTION 170

REJECT PROCESSING SECTION 180

IMAGE BRIGHTNESS DATABASE 171

SIMILARITY CALCULATION SECTION 172

IMAGE-BRIGHTNESS-STATISTIC CALCULATION SECTION 181

SIMILARITY-DIFFERENCE CALCULATION SECTION 182

173 VIEW-TYPE DETERMINATION SECTION

184 REJECT DETERMINATION SECTION

183 THRESHOLD SETTING SECTION

MEASUREMENT-POSITION SETTING PROCESSING SECTION 6

MEASUREMENT CALCULATION SECTION 11

DISPLAY SECTION 12

# FIG.16

220

INPUT IMAGE
INFORMATION

171

| 221 | 222 | 223 | 224 |
|---|---|---|---|
| VIEW TYPE | STANDARD VIEWAL IMAGE INFORMATION | MEASUREMEN T ITEM | MEASUREMENT POSITION |

EP 2 072 013 A1

## FIG.17

41

FIG.18

228

229

# FIG.19

A

CALCULATE SIMILARITY ← S6001

171

THRESHOLD — S6002

S6003

IS SIMILAR VIEW PRESENT?

No

Yes

B

DISPLAY VIEW RECOGNITIION RESULT — S6004

SELECT AND CORRECT VIEW TYPE — S6005

END

# FIG.20

# FIG.21

B

CALCULATE SIMILARITY DIFFERENCE — S7001

THRESHOLD — S7002

S7003 — IS VAGUE VIEW BETWEEN TWO CATEGORIES? — N

Y

S7006 — DISPLAY VIEW RECOGNITIION RESULT

DISPLAY VIEW RECOGNITIION RESULT — S7004

SELECT AND CORRECT VIEW TYPE — S7005

END

FIG.22

FIG.23

EP 2 072 013 A1

253

| PLA | PSA | A2C | A3C | A4C |
|-----|-----|-----|-----|-----|
| 25 | 20 | 10 | 15 | 40 |

250

252

A4C

251

254

SIMILARITY

VIEW TYPE

PLA PSA A2C A3C A4C

# FIG.24

(a)

260

262

261

ITEM A ▼

263

(b)

264

266

265

ITEM B . ▼

267

FIG.25

(A)

↓

INPUT MEASUREMENT ITEM — S8001

↓

DETERMINE VIEW TYPES USED AS CLASSIFICATION CATEGORIES — S8002

↓

S8003

CALCULATE SIMILARITY ← 171

↓

THRESHOLD — S8004

S8005

IS SIMILAR VIEW PRESENT? — No → DISPLAY VIEW RECOGNITIION RESULT — S8006

Yes

↓

(B)

↓

SELECT AND CORRECT VIEW TYPE — S8007

↓

END

FIG.26

# FIG.27

**281** MEASUREMEN T ITEM A

**283** MEASUREMEN T ITEM B

**271** FOUR-CHAMBER

**272** TWO-CHAMBER

**273** LONG-AXIS

**274** SHORT-AXIS BASE LEVEL

**275** SHORT-AXIS MID LEVEL

**276** SHORT-AXIS APEX LEVEL

## INTERNATIONAL SEARCH REPORT

| | International application No. |
|---|---|
| | PCT/JP2007/068156 |

A. CLASSIFICATION OF SUBJECT MATTER
*A61B8/08*(2006.01)i

According to International Patent Classification (IPC) or to both national classification and IPC

B. FIELDS SEARCHED

Minimum documentation searched (classification system followed by classification symbols)
A61B8/08

Documentation searched other than minimum documentation to the extent that such documents are included in the fields searched
Jitsuyo Shinan Koho          1922-1996    Jitsuyo Shinan Toroku Koho   1996-2007
Kokai Jitsuyo Shinan Koho    1971-2007    Toroku Jitsuyo Shinan Koho   1994-2007

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2005-348807 A (Shimadzu Corp.), 22 December, 2005 (22.12.05), | 1,5,6,9,11, 13,19 |
| Y | Par. Nos. [0019] to [0026]; Figs. 3, 4 (Family: none) | 2,3,8,10,20 |
| Y | JP 2004-290404 A (GE Medical Systems Global Technology Co., L.L.C.), 21 October, 2004 (21.10.04), Par. Nos. [0029] to [0039] & US 2004/193053 A1    & DE 102004015477 A1 | 2,8,20 |
| Y | JP 2000-5173 A (GE Yokogawa Medical Systems, Ltd.), 11 January, 2000 (11.01.00), Par. No. [0031] (Family: none) | 3 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 04 October, 2007 (04.10.07) | 16 October, 2007 (16.10.07) |

| Name and mailing address of the ISA/ Japanese Patent Office | Authorized officer |
|---|---|
| Facsimile No. | Telephone No. |

Form PCT/ISA/210 (second sheet) (April 2007)

**INTERNATIONAL SEARCH REPORT**

International application No.

PCT/JP2007/068156

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2006-122686 A (Sonosite, Inc.),<br>18 May, 2006 (18.05.06),<br>Par. Nos. [0102] to [0106]<br>& WO 2005/032375 & EP 1653410 A1<br>& US 2005/96528 A1 | 10 |
| A | JP 2002-306481 A (Olympus Optical Co., Ltd.),<br>22 October, 2002 (22.10.02),<br>Full text; all drawings<br>(Family: none) | 1-20 |
| A | JP 2005-193017 A (General Electric Co.),<br>21 July, 2005 (21.07.05),<br>Full text; all drawings<br>& US 2005/129297 A1 | 1-20 |
| A | JP 2004-229924 A (Aloka System Engineering Co., Ltd.),<br>19 August, 2004 (19.08.04),<br>Full text; all drawings<br>(Family: none) | 1-20 |
| A | JP 2006-127 A (Fuji Photo Film Co., Ltd.),<br>05 January, 2006 (05.01.06),<br>Full text; all drawings<br>(Family: none) | 1-20 |
| A | JP 2004-174220 A (Japan Science and Technology Agency),<br>24 June, 2004 (24.06.04),<br>Full text; all drawings<br>(Family: none) | 1-20 |

Form PCT/ISA/210 (continuation of second sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005224465 A

- JP 2002140689 A